(19) Europäisches Patentamt
European Patent Office
Office européen des brevets

(11) Veröffentlichungsnummer: **0 192 829**
**B1**

(12) **EUROPÄISCHE PATENTSCHRIFT**

(45) Veröffentlichungstag der Patentschrift: **26.09.90**

(21) Anmeldenummer: **85115037.5**

(22) Anmeldetag: **27.11.85**

(51) Int. Cl.$^5$: **C 07 C 233/16,** C 07 C 233/31, C 07 C 235/88, C 07 C 237/20, C 07 C 255/50, C 07 D 303/22, C 07 D 211/22, A 61 K 31/16, A 61 K 31/165, A 61 K 31/275, A 61 K 31/445

# (54) Amino-propanol-Derivate, Verfahren zu ihrer Herstellung und diese Verbindungen enthaltende Arzneimittel sowie Zwischenprodukte.

(30) Priorität: **01.12.84 DE 3443998**

(43) Veröffentlichungstag der Anmeldung:
**03.09.86 Patentblatt 86/36**

(45) Bekanntmachung des Hinweises auf die Patenterteilung:
**26.09.90 Patentblatt 90/39**

(84) Benannte Vertragsstaaten:
**AT BE CH DE FR GB IT LI LU NL SE**

(56) Entgegenhaltungen:
**EP-A-0 034 461**
**EP-A-0 083 256**
**CH-A- 504 408**
**GB-A-2 113 685**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Sandhofer Strasse 116**
**D-6800 Mannheim 31 (DE)**

(72) Erfinder: **Simon, Herbert, Dr. rer. nat.**
**Händelstrasse 5**
**D-6840 Lampertheim (DE)**
Erfinder: **Michel, Helmut**
**Ziegelgasse 2a**
**D-6800 Mannheim 31 (DE)**
Erfinder: **Friebe, Walter-Gunar, Dr. rer. nat.**
**Sophienstrasse 8**
**D-6800 Mannheim 1 (DE)**
Erfinder: **Bartsch, Wolfgang, Dr. med. vet.**
**Franconviller-Strasse 5**
**D-6806 Viernheim (DE)**

**EP 0 192 829 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung sind neue Aminopropanol-Derivate der allgemeinen Formel I

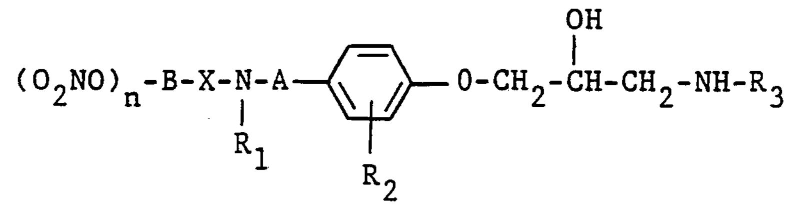

(I)

in welcher

der Rest —B—X—$NR_1$— eine ($C_2$—$C_6$-Alkylen)carbonylamino-, ($C_5$—$C_6$ Cycloalkylen)carbonylamino-, Cyclohexylenmethylcarbonylamino-, Ethylenoxymethylcarbonylamino-, Piperidindiyl- oder Methylenpiperidinyl-Gruppe darstellt, oder für den Fall, daß A eine —CO—$CH_2$-Gruppe bedeutet, —B—X—$NR_1$— auch eine $C_2$—$C_6$-Alkylenaminogruppe bedeuten kann,

A eine direkte Bindung, eine Ethylenkette oder eine —CO—$CH_2$-Gruppe bedeutet,

n die Zahlen 1 bis 3 bedeutet,

$R_2$ Wasserstoff, Cyano, Acetyl oder Ethoxycarbonyl bedeutet und

$R_3$ eine Kohlenwasserstoff- oder Nitroxy-Kohlenwasserstoff-Gruppe mit 3—8 Kohlenstoffatomen bedeutet,

sowie deren physiologisch verträgliche Salze.

Als "Alkylenketten" in der Definition von B koennen, geradkettige oder verzweigte Alkylenreste von 2—6 Kohlenstoffatomen eingesetzt werden. Insbesondere kommen in Frage die Ethylen-, Trimethylen-, Tetramethylen-, Pentamethylen- oder Hexamethylen-Gruppierung bzw. die 1-Methyltrimethylen-, 1,1-Dimethyltrimethylen- oder 2,2-Dimethyltrimethylen-Gruppierung.

Unter einer "Kohlenwasserstoff-Gruppe" bei der Definition von $R^3$ werden geradkettige oder verzweigte, gesaettigte oder ungesaettigte Kohlenwasserstoffreste mit 3—8, vorzugsweise 3—5, Kohlenstoffatomen verstanden. Besonders bevorzugt sind der Isopropyl-, tert.-Butyl-sowie der 2-Methyl-3-butin-2-yl-Rest. Bei "Nitroxy-Kohlenwasserstoff-Gruppen" ist jeweils ein H-Atom der entsprechenden Gruppen durch einen Nitroxy-Rest ersetzt. Besonders bevorzugt ist der 1-Nitroxy-but-3-yl-Rest.

Die erfindungsgemaeßen Verbindungen der Formel I tragen in der Regel eine oder zwei Nitroxy-Gruppen, bevorzugt sind jedoch Mono-Nitroxy-Verbindungen.

Die Verbindungen aehnlicher Art ohne die Gruppierung —O—$NO_2$ sind bereits beschrieben in DE—OS—1 667 883, DE—OS—1 793 511, DE—OS—2 007 751, DE—OS—2 106 209, DE—OS—2 106 816, DE—OS—2 253 776, DE—OS—2 503 222, AT—334 385, S. African 6 803 130 oder S. African 6 808 345.

In der EP—A—34 461 ist u.a. eine 1-Phenoxy-2-hydroxypropylamino-Verbindung beschreiben, die in 4-Stellung am Phenyl-Ring eine 1-Nitroxyalkylamino-Gruppe trägt.

Diese Verbindung zeigt im Vergleich zu den vorliegenden Verbindungen praktisch keine β-blockierenden Eigenschaften und weist eine hohe Denitrierungsgeschwindigkeit auf ($V_{rel}$), d.h. sie besitzt nur eine kurze Wirkdauer.

Die erfindungsgemaeßen Verbindungen der allgemeinen Formel I besitzen wertvolle Eigenschaften. Sie besitzen nicht nur β-Rezeptoren blockierende Aktivitaet, sondern sie bewirken auch eine Verminderung des Sauerstoffbedarfs des Herzens, eine Erhoehung des Blutflusses und eine Erniedrigung des Blutdruckes. Sie eignen sich daher zur Prophylaxe und/oder Behandlung von Herz- und Kreislauferkrankungen, wie z.B. Hochdruck und Angina Pectoris.

Die erfindungsgemaeßen Verbindungen der allgemeinen Formel I koennen in an sich bekannter Weise dadurch hergestellt werden, daß man

a) eine Verbindung der allgemeinen Formel II

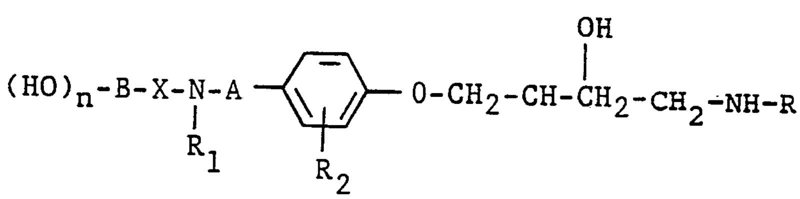

(II)

in der

A, B, X, $R_1$—$R_3$ und n die oben genannten Bedeutungen haben und $R_1$ zusaetzlich noch die Gruppierung —B—$(OH)_n$ darstellen kann,

einer Nitratester-Bildungsreaktion unterwirft, oder

b) eine Verbindung der allgemeinen Formel III

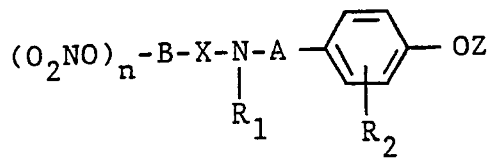 (III)

worin A, B, X, $R_1$, $R_2$ und n die oben angegebenen Bedeutungen besitzen und Z eine der Gruppierungen

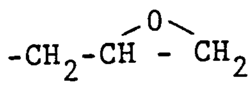 oder 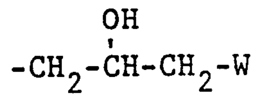

darstellt, worin W eine reaktive Gruppe bedeutet, mit einem Amin der allgemeinen Formel IV

$$H_2N—R_3 \quad \text{(IV)},$$

in der $R_3$ die oben angegebene Bedeutung besitzt, umsetzt, oder

c) eine Verbindung der allgemeinen Formel V

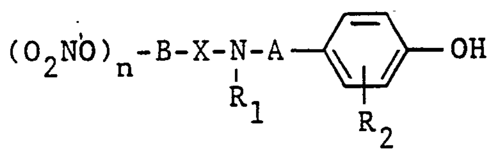 (V)

worin A, B, X, $R_1$, $R_2$ und n die oben angegebenen Bedeutungen haben

c1) mit einer Verbindung der allgemeinen Formel VI

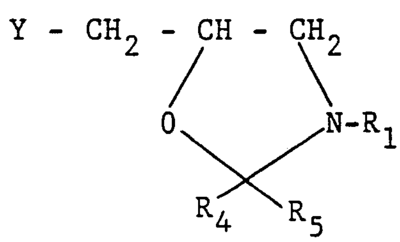 (VI)

worin $R_3$ die oben angegebene Bedeutung hat, Y Mesyloxy, Tosyloxy oder Halogen, $R_4$ Wasserstoff oder Alkyl und $R_5$ unabhaengig Wasserstoff, Alkyl oder Phenyl bedeuten oder $R_4$ und $R_5$ zusammen mit dem benachbarten Kohlenstoffatom ein Carbonyl-Radikal bilden, oder

c2) mit einer Verbindung der allgemeinen Formel VII

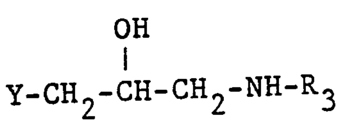 (VII)

worin Y und $R_3$ die oben angegebenen Bedeutungen haben, umsetzt, oder

d) eine Verbindung der allgemeinen Formel VIII

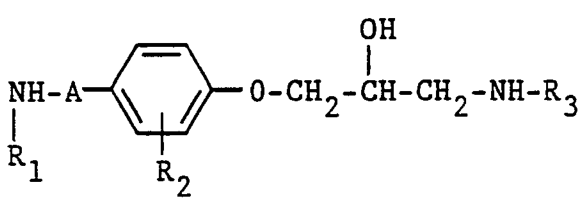 (VIII)

in der A und $R_1$—$R_3$ die oben angegebenen Bedeutungen haben, mit einer Verbindung der allgemeinen Formel IX

$$(O_2NO)_n—B—X—W \quad (IX),$$

wobei B, W, X und n die oben angegebenen Bedeutungen haben, umsetzt, oder

e) eine Verbindung der allgemeinen Formel X

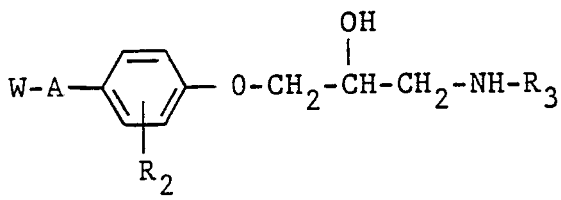

(X)

in der A, $R_2$, $R_3$ und W die oben genannten Bedeutungen haben, mit einer Verbindung der allgemeinen Formel XI

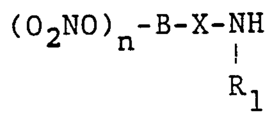

(XI)

worin B, $R_1$, X und n die oben genannten Bedeutungen haben, umsetzt und die so erhaltenen Verbindungen gewuenschtenfalls in ihre physiologisch vertraeglichen Salze ueberfuehrt.

Die Verbindungen der allgemeinen Formel II (Zwischenprodukte fuer die Herstellung von Verbindungen der allgemeinen formel I) sind ebenfalls neu und Gegenstand dieser Anmeldung. Sie koennen hergestellt werden, indem man in an sich bekannter Weise eine Verbindung der allgemeinen Formel XII

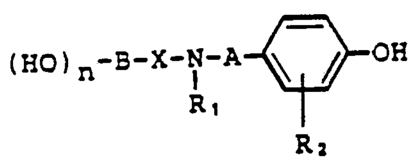

(XII),

in der A, B, $R_1$, $R_2$, X und n die oben genannten Bedeutungen besitzen,

a) in einer Veretherungsreaktion zu einer Verbindung der allgemeinen Formel XIII

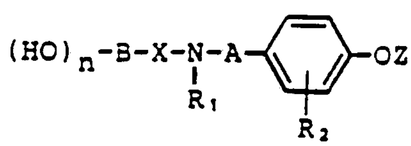

(XIII),

in der A, B, $R_1$, $R_2$, Z, X und n die oben angegebene Bedeutung haben, umsetzt und diese durch Umsetzung mit einem Amin der allgemeinen Formel IV in die Verbindungen der allgemeinen Formel II ueberfuehrt,

b1) mit einer Verbindung der allgemeinen Formel VI oder

b2) mit einer Verbindung der allgemeinen Formel VII umsetzt, oder

c) eine Verbindung der allgemeinen Formel VIII mit einer Verbindung der allgemeinen Formel XIV

$$(HO)_n—B—X—W \quad (XIV),$$

in der B, W, X und n die oben angegebenen Bedeutungen besitzen, umsetzt, oder

d) eine Verbindung der allgemeinen Formel X mit einer Verbindung der allgemeinen Formel XV

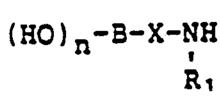

(XV),

in der B, $R_1$, X und n die oben angegebenen Bedeutungen haben, umsetzt.

Die Verbindungen der allgemeinen Formel III (Zwischenprodukte zur Herstellung von Verbindungen der allgemeinen formel I) sind ebenfalls neu und Gegenstand dieser Anmeldung.

Sie koennen hergestellt werden, indem man in an sich bekannter Weise

a) eine Verbindung der allgemeinen Formel XIII einer Nitratester-Bildungsreaktion unterwirft, oder

b) eine Verbindung der allgemeinen Formel V einer Glycidether- oder Halogenhydrin-Bildungsreaktion unterwirft, oder

c1) eine Verbindung der allgemeinen Formel XVI

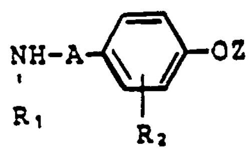

(XVI),

worin A, $R_1$, $R_2$ und Z die oben angegebenen Bedeutungen haben, mit einer Verbindung der allgemeinen Formel IX umsetzt, oder

c2) eine Verbindung der allgemeinen Formel XVII

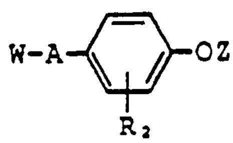

(XVII),

worin A, $R_2$, W und Z die oben angegebenen Bedeutungen besitzen, mit einer Verbindung der allgemeinen Formel XI umsetzt, oder

d) Verbindungen der allgemeinen Formel XVIII

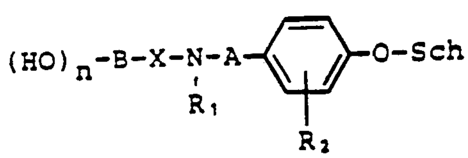

(XVIII),

worin A, B, $R_1$, $R_2$, X und n die oben angegebenen Bedeutungen besitzen und Sch eine Schutzgruppe wie Alkoxycarbonyl oder Tetrahydropyranyl darstellt, einer Nitratester-Bildungsreaktion unterwirft, anschließend die Schutzgruppe abspaltet und die so erhaltenden Verbindungen den allgemeinen Formel V einen Glycidether- oder Halogenhydrin-Bildungsreaktion unterwirft.

Die Verbindungen der allgemeinen Formel V (Zwischenprodukte fuer die Herstellung von Verbindungen der allgemeinen Formel I) koennen hergestellt werden, in dem man in an sich bekannter Weise

a) eine Verbindung der allgemeinen Formel XII einer Nitratester-Bildungsreaktion unterwirft, oder

b) eine Verbindung der allgemeinen Formel XIX

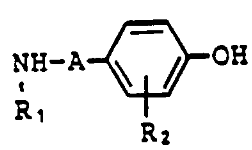

(XIX),

worin A, $R_1$ und $R_2$ die oben angegebenen Bedeutungen besitzen, mit einer Verbindung der allgemeinen Formel IX umsetzt, oder

c) eine Verbindung der allgemeinen Formel XX

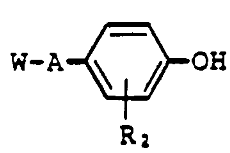

(XX),

worin A, $R_2$ und W die oben angegbenen Bedeutungen besitzen, mit einer Verbindung der allgemeinen Formel XI umsetzt.

Die Nitratester-Bildungsreaktion der Verbindungen der allgemeinen Formeln II, XII, XIII, XV und XVIII

kann durchgefuehrt werden, indem man die Verbindungen der allgemeinen Formeln II, XII, XIII, XV und XVIII mit einem nitratesterbildenden Reagenz, wie rauchender Salpetersaeure, einer Mischung aus rauchender Salpetersaeure und Acetanhydrid oder einer Mischung aus rauchender Salpetersaeure und konz. Schwefelsaeure bei niedrigen Temperaturen in Gegenwart oder Abwesenheit eines inerten Loesungsmittels umsetzt.

Die Reaktionstemperaturen liegen zwischen Raumtemperatur und −60°C, bevorzugt zwischen −10°C und −30°C. Das Molverhaeltnis der Reaktionspartner liegt zwischen 1 und 10.

Alternativ kann die Nitratester-Bildungsreaktion durchgefuehrt werden, indem man in einer Verbindung der allgemeinen Formeln II, XII, XIII oder XVIII selektiv eine aliphatische Hydroxylgruppe halogeniert und anschließend das Reaktionsprodukt mit Silbernitrat in Gegenwart oder Abwesenheit eines Loesungsmittels bei Temperaturen zwischen Raumtemperatur und 100°C umsetzt. Das Molverhaeltnis der Umsetzungsreaktion zwischen der Halogenverbindung und Silbernitrat kann zwischen 1 und 10 liegen.

Die Halogenierungsreaktion kann nach literaturbekannten Verfahren durchgefuehrt werden, indem man eine Verbindung der allgemeinen Formeln II, XII, XIII oder XVIII mit Mesylchlorid oder Tosylchlorid in Gegenwart eines saeurebindenden Mittels umsetzt und das erhaltene Reaktionsprodukt anschließend mit einem Alkalihalogenid in einem organischen Loesungsmittel, z.B. Dimethylformamid, zur Reaktion bringt.

Die Herstellung der Verbindungen der allgemeinen Formeln III, XIII und XVI erfolgt in an sich bekannter Weise dadurch, daß man Epihalogenhydrine in einem organischen Loesungsmittel und/oder Wasser in Gegenwart eines saeurebindenden Mittels, wie Alkalihydroxide und -hydride oder organische Stickstoffbasen, mit Verbindungen der allgemeinen Formeln V, XII bzw. XXI

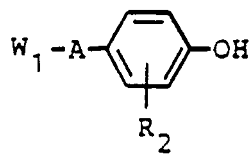

(XXI),

worin bei Formel XXI A die oben angegebene Bedeutung hat und $W_1$ eine $NO_2$-Gruppe oder eine Alkylcarbonylamino-Gruppe darstellt, bei Temperaturen zwischen Raumtemperatur und 100°C umsetzt und gegebenenfalls die Gruppe $W_1$ in die Gruppe

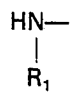

ueberfuehrt. Die Molverhaeltnisse der Verbindungen der allgemeinen Formeln V, XII und XXI zu den Epihalogenhydrinen koennen zwischen 1 und 100 liegen.

Die Umsetzung der Verbindungen mit den allgemeinen Formeln V, XII und XXI mit einer Verbindung der allgemeinen Formel VI oder VII erfolgt in einem organischen Loesungsmittel wie Methanol, Ethanol, Propanol, Benzol, Toluol oder Dimethylformamid bei Temperaturen zwischen Raumtemperatur und 100°C. Das Molverhaeltnis kann zwischen 1 und 10 betragen.

Die Umsetzungen der Verbindungen der allgemeinen Formeln III, XIII und XVI mit einem Amin der allgemeinen Formel IV erfolgen in Gegenwart oder Abwesenheit eines Loesungsmittels zwischen 0°C und 90°C, bevorzugt zwischen 20°C und 50°C. Als Loesungsmittel koennen z.B. Methanol, Ethanol, Propanol, iso-Propanol, Benzol, Toluol oder Dimethylformamid verwendet werden. Das molare Verhaeltnis der Reaktionspartner ist nicht kritisch. Es koennen Verhaeltnisse zwischen 2 und 100 gewaehlt werden.

Die Umsetzungen der Amine VIII, XVI und XIX mit aktivierten Verbindungen der allgemeinen Formeln IX und XIV werden in einem organischen Loesungsmittel wie Hexan, Diethylether, Tetrahydrofuran, Methylenchlorid, Benzol, Toluol oder Dimethylformamid oder in waessriger Loesung bei Temperaturen zwischen −50°C und +70°C, bevorzugt zwischen −30°C und Raumtemperatur, durchgefuehrt.

Die aktivierten Verbindungen der allgemeinen Formeln IX und XIV koennen dabei z.B. in Form von Carbonsaeurehalogeniden, -anhydriden oder -estern vorliegen, oder die Aktivierung der Carbonsaeuren erfolgt durch aktivierende Reagenzien wie N,N'-Carbonyldiimidazol, N,N'-Dicyclohexylcarbodiimide, 1-Ethyl-3-(3-dimethylaminopropyl)carbodiimid, 1-Alkyl-2-halogen-pyridiniumsalze o.ä. Die Aktivierung und die Umsetzung mit den Aminen VIII, XVI und XIX kann dabei in einem Syntheseschritt durchgefuehrt werden. Es koennen molare Verhaeltnisse zwischen 1 und 10 gewaehlt werden.

Die Umsetzungen der Amine XI und XV mit aktivierten Verbindungen der allgemeinen Formeln X, XVII und XX werden in einem organischen Loesungsmittel wie Ether, Tetrahydrofuran, Dioxan, Methylenchlorid, Benzol, Tuluol oder Dimethylformamid oder in Wasser bei Temperaturen zwischen −50°C und +100°C, bevorzugt zwischen −30°C und Raumtemperatur, durchgefuehrt. Die aktivierten Verbindungen der allgemeinen Formeln X, XVII und XX koennen dabei z.B. in Form von Carbonsaeurehalogeniden, -anhydriden und -estern vorliegen, oder die Aktivierung erfolgt im Reaktionsgemisch mit aktivierenden Reagenzien wie N,N'-Carbonyldiimidazol, N,N'-Dicyclohexylcarbodiimid, oder 1-Ethyl-3-(3-dimethylaminopropyl)carbondiimid. Die Aktivierung und die Umsetzung mit den Aminen XI und XV kann dabei in einem Schritt erfolgen. Das molare Verhaeltnis der Reaktionspartner ist nicht kritisch. Es koennen Verhaeltnisse zwischen 1 und 10 gewaehlt werden.

Die erfindungsgemaeßen Verbindungen besitzen asymmetrische Kohlenstoffatome.

Gegenstand der Erfindung sind daher auch saemtliche moeglichen Diastereomerengemische, Racemate und saemtliche optisch aktiven Formen der erfindungsgemaeßen verbindungen der allgemeinen Formeln I, II, III und gegebenenfalls V.

Zur Ueberfuehrung der Verbindungen der allgemeinen Formeln I und II in ihre pharmakologisch unbedenklichen Salze setzt man diese, vorzugsweise in einem organischen Loesungsmittel, mit der aequivalenten Menge einer anorganischen oder organischen Saeure, z.B. Salzsaeure, Bromwasserstoffsaeure, Salpetersaeure, Phosphorsaeure, Schwefelsaeure, Ameisensaeure, Essigsaeure, Propionsaeure, Oxalsaeure, Fumarsaeure, Maleinsaeure, Bernsteinsaeure, Adipinsaeure, Benzoesaeure, Salicylsaeure, o-Acetoxybenzoesaeure, Zimtsaeure, Naphthoesaeure, Mandelsaeure, Citronensaeure, Aepfelsaeure, Weinsaeure, Asparaginsaeure, Glutaminsaeure, Methansulfonsaeure oder p-Toluolsulfonsaeure um.

Die erfindungsgemaeßen neuen Substanzen I und II und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt. Derartige Zusaetze sind z.B. Tartrat- und Citratpuffer, Ethanol, Komplexbildner (wie Ethylendiamintetraessigsaeure und deren nicht toxische Salze), hochmolekulare Polymere (wie fluessiges Polyethylenoxid) zur Viskositaetsregulierung. Fest Traegerstoffe sind z.B. Staerke, Lactose, Mannit, Methylcellulose, Talkum, hochdisperse Kieselgelsaeuren, hoehermolekulare Fettsaeuren (wie Stearinsaeure), Gelatine, Agar-Agar, Calciumphosphat, Magnesiumstearat, tierische und pflanzliche Fette und feste hochmolekulare Polymere (wie z.B. Polyethylenglykole), fuer orale Applikation geeignete Zubereitungen koennen gewuenschtenfalls Geschmacks- und Sueßstoffe enthalten.

Die erfindungsgemaeßen Verbindungen werden ueblicherweise in Mengen von 20—500 mg pro Tag bezogen auf 75 kg Koerpergewicht appliziert. Bevorzugt ist es, 2—3 mal pro Tag 1—2 Tabletten mit einem Wirkstoffgehalt von 10—200 mg zu verabreichen. Die Tabletten koennen auch retardiert sein, wodurch nur noch 1 mal pro Tag 1—2 Tabletten mit 20—500 mg Wirkstoff gegeben werden muß. Der Wirkstoff kann auch durch Injektion 1—8 mal pro Tag bzw. durch Dauerinfusion gegeben werden, wobei Mengen von 5—200 mg/Tag normalerweise ausreichen.

Neben den nachfolgend aufgefuehrten Beispiel sind insbesondere die folgenden Verbindungen im Sinne der Anmeldung bevorzugt:

1-[4-(2-Methyl-1-nitroxy-2-propyl)carbonylamino]phenoxy-3-isopropylamino-2-propanol

3-(2-Methyl-3-butin-2-yl)amino-1-[4-(2-methyl-1-nitroxy-2-propyl)carbonylamino]phenoxy-2-propanol

3-Isopropylamino-1-[4-[[(t-4-nitroxy)cyclohexyl]-r-1-carbonylamino]]phenoxy-2-propanol

3-(2-Methyl-3-butin-2-yl)amino-1-[4-[[(t-4-nitroxy)cyclohexyl]-r-1-carbonylamino]]phenoxy-2-propanol

3-Isopropylamino-1-[4-[[(t-2-nitroxy)cyclohexyl]-r-1-methylcarbonylamino]]phenoxy-2-propanol

3-(2-Methyl-3-butin-2-yl)amino-1-[4-[[(t-2-nitroxy)cyclohexyl]-r-1-methylcarbonylamino]]phenoxy-2-propanol

1-[2-Cyano-4-(3-nitroxypropyl)carbonylamino]phenoxy-3-isopropylamino-2-propanol

1-[2-Cyano-4-(3-nitroxypropyl)carbonylamino]phenoxy-3-(2-methyl-3-butin-2-yl)amino-2-propanol

1-[2-Cyano-4-[[(t-4-nitroxy-)cyclohexyl]-r-1-carbonylamino]]phenoxy-3-isopropylamino-2-propanol

1-[2-Cyano-4-[[(t-4-nitroxy)cyclohexyl]-r-1-carbonylamino]]phenoxy-3-(2-methyl-3-butin-2-yl)amino-2-propanol

3-Isopropylamino-1-[2-methylcarbonyl-4-(3-nitroxypropyl)carbonylamino]phenoxy-2-propanol

3-(2-Methyl-3-butin-2-yl)amino-1-[2-methylcarbonyl-4-(3-nitroxypropyl)carbonylamino]phenoxy-2-propanol

3-Isopropylamino-1-[2-methylcarbonyl-4-[[(t-4-nitroxy)cyclohexyl]-r-1-carbonylamino]phenoxy-2-propanol

3-(2-Methyl-3-butin-2-yl)amino-1-[2-methylcarbonyl-4-[[(t-nitroxy)cyclohexyl]-r-1-carbonylamino]]phenoxy-2-propanol

1-[2-Ethoxycarbonyl-4-(4-nitroxybutyl)carbonylamino]phenoxy-3-tert.-butylamino-2-propanol

1-[2-Ethoxycarbonyl-4-(2-methyl-1-nitroxy-2-propyl)carbonylamino]phenoxy-3-tert.-butylamino-2-propanol

1-[2-Ethoxycarbonyl-4-[[(t-4-nitroxy)cyclohexyl-r-1-carbonylamino]]phenoxy-3-tert.-butylamino-2-propanol

1-[2-Ethoxycarbonyl-4-[[[(t-2-nitroxy)cyclohexyl]-r-1-methyl]carbonyl]amino]phenoxy-3-tert.-butylamino-2-propanol

1-[2-Ethoxycarbonyl-4-(3-nitroxypropyl)carbonylamino]phenoxy-3-isopropylamino-2-propanol

1-[2-Ethoxycarbonyl-4-(3-nitroxypropyl)carbonylamino]phenoxy-3-(2-methyl-3-butin-2-yl)amino-2-propanol

1-[4-[(2-Nitroxyethyl)amino]carbonylmethyl]phenoxy-3-tert.-butylamino-2-propanol

1-[4-[(1-Nitroxy-but-3-yl)amino]carbonylmethyl]phenoxy-3-tert.-butylamino-2-propanol

1-[4-[(2-Methyl-4-nitroxy-2-butyl)amino]carbonylmethyl]phenoxy-3-tert.-butylamino-2-propanol

1-[4-[(2,3-Dinitroxy-propyl)amino]carbonylmethyl]phenoxy-3-tert.-butylamino-2-propanol
1-[4-[N,N-Bis(2-nitroxyethyl)amino]carbonylmethyl]phenoxy-3-tert.-butylamino-2-propanol
1-[4-[N,N-Bis(2-nitroxypropyl)amino]carbonylmethyl]phenoxy-3-tert.-butylamino-2-propanol
trans-1-[4-[(4-Nitroxycyclohexyl)amino]carbonylmethyl]phenoxy-3-tert.-butylamino-2-propanol
3-Isopropylamino-1-[4-[(3-nitroxypropyl)amino]carbonylmethyl]phenoxy-2-propanol
3-Isopropylamino-1-[4-[(2-nitroxypropyl)amino]carbonylmethyl]phenoxy-2-propanol
3-Isopropylamino-1-[4-[(1-nitroxy-but-3-yl)amino]carbonylmethyl]phenoxy-2-propanol
3-Isopropylamino-1-[4-[(2-methyl-4-nitroxy-2-butyl)amino]carbonylmethyl]phenoxy-2-propanol
3-Isopropylamino-1-[4-[(2,2-dimethyl-3-nitroxypropyl)amino]carbonylmethyl]phenoxy-2-propanol
3-Isopropylamino-1-[4-[(2,3-dinitroxy-propyl)amino]carbonylmethyl]phenoxy-2-propanol
3-Isopropylamino-1-[4-[[N-methyl-N-(3-nitroxypropyl)]amino]carbonylmethyl]phenoxy-2-propanol
3-Isopropylamino-1-[4-[[N-methyl-N-(2-nitroxypropyl)]amino]carbonylmethyl]phenoxy-2-propanol
3-Isopropylamino-1-[4-[[N-methyl-N-(4-nitroxybutyl)]amino]carbonylmethyl]phenoxy-2-propanol
3-Isopropylamino-1-[4-[[N-methyl-N-(1-nitroxy-but-3-yl)]amino]carbonylmethyl]phenoxy-2-propanol
3-Isopropylamino-1-[4-[[N-methyl-N-(2,2-dimethyl-3-nitroxypropyl)]amino]carbonylmethyl]phenoxy-2-propanol
trans-3-Isopropylamino-1-[4-[(2-nitroxycyclohexyl)amino]carbonylmethyl]phenoxy-2-propanol
trans-3-Isopropylamino-1-[4-[(4-nitroxycyclohexyl)amino]carbonylmethyl]phenoxy-2-propanol
cis-3-Isopropylamino-amino-1-[4-[(4-nitroxycyclohexyl)amino]carbonylmethyl]phenoxy-2-propanol
trans-3-Isopropylamino-1-[4-[[(4-nitroxycyclohexyl)methyl]amino]carbonylmethyl]phenoxy-2-propanol
trans-3-Isopropylamino-[4-[[(2-nitroxycyclohexyl)methyl]amino]carbonylmethyl]phenoxy-2-propanol
3-Isopropylamino-1-[4-(4-nitroxy-1-piperidino)carbonylmethyl]phenoxy-2-propanol
3-Isopropylamino-1-[4-(2-nitroxymethyl-1-piperidino)carbonylmethyl]phenoxy-2-propanol
3-Isopropylamino-1-[4-(3-nitroxymethyl-1-piperidino)carbonylmethyl]phenoxy-2-propanol
3-(2-Methyl-3-butin-2-yl)amino-1-[4-[(3-nitroxypropyl)amino]carbonylmethyl]phenoxy-2-propanol
3-(2-Methyl-3-butin-2-yl)amino-1-[4-[(2-nitroxypropyl)amino]carbonylmethyl]phenoxy-2-propanol
3-(2-Methyl-3-butin-2-yl)amino-1-[4-(4-nitroxy-1-piperidino)carbonylmethyl]phenoxy-2-propanol
1-[4-(3-Nitroxypropyl)carbonylamino]phenoxy-3-isopropylamino-2-propanol
1-[4-(3-Nitroxypropyl)carbonylamino]phenoxy-3-(2-methyl-3-butin-2-yl)amino-2-propanol
1-[4-[2-(3-Nitroxypropyl)carbonylamino]ethyl]phenoxy-3-tert.-butylamino-2-propanol
1-[4-[2-(2-Methyl-1-nitroxy-2-propyl)carbonylamino]ethyl]phenoxy-3-tert.-butylamino-2-propanol
1-[4-[[N-Methyl-N-(3-nitroxypropyl)]amino]carbonylmethyl]phenoxy-3-tert.-butylamino-2-propanol
1-[4-[[N-Methyl-N-(2-nitroxypropyl)]amino]carbonylmethyl]phenoxy-3-tert.-butylamino-2-propanol
1-[4-[[N-Methyl-N-(4-nitroxybutyl)]amino]carbonylmethyl]phenoxy-3-tert.-butylamino-2-propanol
1-[4-[[N-Methyl-N-(1-nitroxy-but-3-yl)]amino]carbonylmethyl]phenoxy-3-tert.-butylamino-2-propanol
cis-1-[4-[(4-Nitroxycyclohexyl)amino]carbonylmethyl]phenoxy-3-tert.-butylamino-2-propanol
1-[4-(2-Nitroxymethyl-1-piperidino)-carbonylmethyl]phenoxy-3-tert.-butylamino-2-propanol
1-[4-[(2-Nitroxyethylmercapto)methylcarbonylamino]phenoxy]-3-tert.-butylamino-2-propanol
1-[4-[(2-Nitroxyethylsulfinyl)methylcarbonylamino]phenoxy]-3-tert.-butylamino-2-propanol
1-[4-[(2-Nitroxyethylsulfonyl)methylcarbonylamino]phenoxy-3-tert.-butylamino-2-propanol.

### Beispiel 1

1-[4-(3-Nitroxypropyl)carbonylamino]phenoxy-3-tert.-butylamino-2-propanol hydrochlorid

Zu einer Loesung von 5.0 g 1-(4-Amino)phenoxy-3-tert.-butylamino-2-propanol in 100 ml trockenem THF wird bei Raumtemperatur eine Loesung von 3.6 g 4-Nitroxybutanoylchlorid getropft. Man ruehrt noch 1 h bei Raumtemperatur und dampft im Vakuum zur Trockne ein. Der verbleibende Rueckstand wird mit wenig Diethylether verrieben. Der erhaltene Niederschlag wird abgesaugt und getrocknet.

Man erhaelt 7.1 g (83% d.Th.) Kristalle vom Schmp. 137—140°C.

Das als Ausgangsmaterial benoetigte 4-Nitroxybutanoylchlorid wird wie folgt hergestellt:

Zu einer Loesung von 1.49 g 4-Nitroxybuttersaeure in 150 ml trockenem Methylenchlorid werden bei Raumtemperatur 2.08 g Phosphorpentachlorid gegeben. Man ruehrt noch 1 h und zieht dann das Loesungsmittel im Vakuum ab. Der verbliebende Rueckstand (1.7 g Oel) wird direkt weiter umgesetzt.

Die als Ausgangsmaterialien benoetigten Verbindungen 1-(4-Amino)phenoxy-3-tert.-butylamino-2-propanol, 1-(4-Amino)phenoxy-3-isopropylamino-2-propanol bzw. 1-(4-Amino)phenoxy-3-(2-methyl-3-butin-2-yl)amino-2-propanol werden nach en sich bekannten Verfahren und gemaeß den Vorschriften in EP 29 992 hergestellt.

Die als Ausgangsmaterial benoetigte 4-Nitroxybuttersaeure wurde nach an sich bekannter Weise aus Natrium-(4-hydroxy)butanoat und einem Gemisch aus 100 proz. Salpetersaeure/96 proz. Schwefelsaeure hergestellt. Die uebrigen Nitroxyalkylcarbonsaeuren wurden analog aus den entsprechenden Hydroxyalkylcarbonsaeuren hergestellt.

### Beispiel 2

In analoger Weise, wie in Beispiel 7 beschrieben, erhaelt man aus 1-(4-Amino)phenoxy-3-tert.-butylamino-2-propanol, 1-(4-Amino)phenoxy-3-isopropylamino-2-propanol bzw. 1-(4-Amino)phenoxy-3-(2-methyl-3-butin-2-yl)amino-2-propanol und den entsprechenden Nitroxyalkylcarbonsaeurechloriden folgende Verbindungen:

| | Bezeichnung | Ausb. % | Schmelzpunkt °C (Lösungsmittel) |
|---|---|---|---|
| a) | 1-[4-(4-Nitroxybutyl)carbonylamino]-phenoxy-3-tert.-butylamino-2-propanol fumarat<br>aus<br>5-Nitroxypentanoylchlorid | 41 | 128—130 (Ethylacetat) |
| b) | 1-[4-(2-Methyl-1-nitroxy-2-propyl)-carbonylamino]phenoxy-3-tert.-butylamino-2-propanol hydrochlorid<br>aus<br>2,2-Dimethyl-3-nitroxy-propionylchlorid (hellgelbes Oel) | 78 | 149—150 (Ethylacetat) |
| c) | 1-[4-[1,1-Bis(nitroxymethyl)ethyl]carbonylamino]phenoxy-3-tert.-butylamino-2-propanol hydrochlorid<br>aus<br>2,2-Bis(nitroxymethyl)propionylchlorid (farbloses Oel) | 90 | 97—100 (Diethylether) |
| d) | 1-[4-[[(t-3,t-4-Dinitroxy)cyclopentyl]-r-1-carbonylamino]]phenoxy-3-tert.-butylamino-2-propanol fumarat<br>aus<br>(t-3,t-4-Dinitroxy)cyclopentancarbonsaeurechlorid (farbloses Oel) | 72 | 198—199 (Diethylether) |

Beispiel 2 (Fortsetzung)

| | Bezeichnung | Ausb. % | Schmelzpunkt °C (Lösungsmittel) |
|---|---|---|---|
| e) | 1-[4-[[(t-4-Nitroxy)cyclohexyl]-r-1-carbonylamino]]phenoxy-3-tert.butylamino-2-propanol hydrochlorid<br>aus<br>trans-4-Nitroxycyclohexancarbonsaeurechlorid (hellgelbes Oel) | 97 | 104—106 (Diethylether) |
| f) | 1-[4-[[(t-3,c-4-Dinitroxy)cyclohexyl]-r-1-carbonylamino]]phenoxy-3-tert.-butylamino-2-propanol hydrochlorid<br>aus<br>(t-3,c-4-Dinitroxy)cyclohexancarbonsaeure-chlorid (farbloses Oel) | 100 | 77—80 (Diethylether) |
| g) | 1-[4-[[(t-2-Nitroxy)cyclohexyl]-r-1-methylcarbonylamino]]phenoxy-3-tert.-butylamino-2-propanol hydrochlorid<br>aus<br>trans-2-(2-Nitroxycyclohexyl)essigsaeure-chlorid (farbloses Oel) | 57 | 115—118 (Ethylacetat) |
| h) | 1-[4-[[(2-Nitroxymethoxy)methyl]-carbonyl-amino]phenoxy]-3-tert.-butylamino-2-propanol-fumarat<br>aus<br>(2-Nitroxyethoxy)acetylchlorid | 32 | 166—167 (Ethylacetat) |

Beispiel 3

1-[2-Cyano-4-(2-methyl-1-nitroxy-2-propyl)carbonylamino]phenoxy-3-tert.-butylamino-2-propanol benzoat

Zu einer Suspension von 2.6 g 1-(4-Amino-2-cyano)phenoxy-3-tert.-butylamino-2-propanol in 50 ml trockenem Methylenchlorid tropft man bei 5°C eine Loesung von 2.0 g 2,2-Dimethyl-3-nitroxy-propionylchlorid in 50 ml trockenem Methylenchlorid, reuhrt weitere 2 h bei 5°C und laeßt ueber Nacht auf Raumtemperatur kommen. Man versetzt mit 100 ml waeßriger Kaliumcarbonatloesung und extrahiert 5 mal mit je 100 ml Methylenchlorid. Die Extrakte werden ueber Natriumsulfat getrocknet. Der Rueckstand wird in Ethylacetat/Diethylether geloest und mit waeßriger Milchsaeureloesung extrahiert, die waeßrige Phase dann 3 mal mit Ethylacetat extrahiert. Die vereinigten organischen Phasen werden ueber Natriumsulfat getrocknet. Der nach dem Eindampfen verbleibende Rueckstand (3.5 g) wird in Ethylacetat geloest und mit 0.8 g Benzoesaeure versetzt. Der erhaltene Niederschlag wird abgesaugt und getrocknet.

Man erhaelt 3.2 g (61% d.Th.) Kristalle vom Schmelzpunkt 131—133°C.

Die als Ausgangsmaterial benoetigte Verbindung 1-(4-Amino-2-cyano)phenoxy-3-tert.-butylamino-2-propanol wird nach an sich bekannten Verfahren und gemaeß den Vorschriften in EP 29 992 hergestellt.

Beispiel 4

In analoger Weise, wie in Beispiel 3 beschrieben, erhaelt man aus 1-(4-Amino-2-cyano)phenoxy-3-tert.-butylamino-2-propanol und Nitroxyalkylcarbonsaeurechloriden folgende Verbindungen:

| | Bezeichnung | Ausb. % | Schmelzpunkt °C (Lösungsmittel) |
|---|---|---|---|
| a) | 1-[2-Cyano-4-(3-Nitroxypropyl)-carbonylamino]-phenoxy-3-tert.-butylamino-2-propanol hydrochlorid<br>aus<br>4-Nitroxybutanoylchlorid | 63 | amorph |
| b) | 1-[2-Cyano-4-[1,1-bis(nitroxymethyl)ethyl]-carbonylamino]phenoxy-3-tert.-butylamino-2-propanol benzoat<br>aus<br>2,2-Bis(nitroxymethyl)propionylchlorid | 40 | 50 (Diethylether) |
| c) | 1-[2-Cyano-4-[[(t-4-nitroxy)-cyclohexyl]-r-1-carbonylamino]]phenoxy-3-tert.-butylamino-2-propanol benzoat<br>aus<br>trans-4-Nitroxycyclohexancarbonsaeurechlorid | 73 | 154—156 (Ethylacetat) |
| d) | 1-[2-Cyano-4-[[(t-3,c-4-dinitroxy)-cyclohexyl]-r-1-carbonylamino]]-phenoxy-3-tert.-butylamino-2-propanol benzoat<br>aus<br>(t-3,c-4-Dinitroxy)cyclohexancarbonsaeurechlorid | 38 | 148 (Ethylacetat) |
| e) | 1-[2-Cyano-4-[[(t-2-nitroxy)cyclohexyl]-r-1-methylcarbonylamino]]phenoxy-3-tert.-butylamino-2-propanol benzoat<br>aus<br>trans-2-(2-Nitroxycyclohexyl)essigsaeurechlorid | 60 | 148—149 (Ethylacetat) |

Beispiel 5

1-[4-(2-Methyl-1-nitroxy-2-propyl)carbonylamino-2-methylcarbonyl]phenoxy-3-tert.-butylamino-2-propanol hydrochlorid

Zu einer Loesung von 3.4 g 1-(4-Amino-2-methylcarbonyl)phenoxy-3-tert.-butylamino-2-propanol in 70 ml trockenem Tetrahydrofuran tropft man bei Raumtemperatur eine Loesung von 2.7 g 2,2-Dimethyl-3-nitroxy-propionylchlorid in 70 ml trockenem Tetrahydrofuran. Man laeßt ueber Nacht ruehren, saugt das kristallisierte Salz ab und trocknet.

Man erhaelt 4.9 g (88% d.Th.) Kristalle vom Schmelzpunkt 175—177°C.

Die als Ausgangsmaterial benoetigte Verbindung 1-(4-Amino-2-methylcarbonyl)phenoxy-3-tert.-butylamino-2-propanol wird nach an sich bekannten Verfahren und gemaeß den Vorschriften in EP 29 992 hergestellt.

Beispiel 6

In analoger Weist, wie in Beispiel 5 beschrieben, erhaelt man aus 1-(4-Amino-2-methylcarbonyl)phenoxy-3-tert.-butylamino-2-propanol und Nitroxyalkylcarbonsäurechloriden folgende Verbindungen:

| | Bezeichnung | Ausb. % | Schmelzpunkt °C (Lösungsmittel) |
|---|---|---|---|
| a) | 1-[2-Methylcarbonyl-4-(3-nitroxypropyl)carbonylamino]phenoxy-3-tert.-butylamino-2-propanol fumarat<br>aus<br>4-Nitroxybutyanoylchlorid | 63 | 63—65 amorph (Ethylacetat) |
| b) | 1-[4-[1,1-Bis(nitroxymethyl)-ethyl]carbonylamino-2-methyl-carbonyl]phenoxy-3-tert.-butylamino-2-propanol hydrochlorid<br>aus<br>2,2-Bis(nitroxymethyl)propionylchlorid | 69 | 141—142 (Ethylacetat) |
| c) | 1-[2-Methylcarbonyl-4-[[(t-4-nitroxy)cyclohexyl]-r-1-carbonylamino]]phenoxy-3-tert.-butylamino-2-propanol hydrochlorid<br>aus<br>trans-4-Nitroxycyclohexancarbonsaeurechlorid | 91 | 135—137 (Tetrahydrofuran) |
| d) | 1-[4-[[(t-3,c-4-Dinitroxy)cyclohexyl]-r-1-carbonylamino]-2-methylcarbonyl]phenoxy-3-tert.-butylamino-2-propanol hydrochlorid<br>aus<br>(t-3,c-4-Dintroxy)cyclohexancarbonsaeurechlorid | 97 | 165—167 (Tetrahydrofuran) |
| e) | 1-[1-Methylcarbonyl-4-[[(t-2-nitroxy)cyclohexyl]-r-1-methylcarbonylamino]]phenoxy-3-tert.-butylamino-2-propanol hydrochlorid<br>aus<br>trans-2-(2-Nitroxycyclohexyl)essigsaeurechlorid | 66 | 132—134 (Aceton) |

Beispiel 7

1-[2-Ethoxycarbonyl-4-(3-nitroxypropyl)carbonylamino]phenoxy-3-tert.-butylamino-2-propanol cyclaminat

Zu einer Lösung von 6.85 g 1-(4-Amino-2-ethoxycarbonyl)phenoxy-3-tert.-butylamino-2-propanol in 250 ml trockenem Tetrahydrofuran gibt man 3 ml Triethylamin und tropft bei 10°C eine Lösung von 3.6 g 4-Nitroxy-butanoylchlorid in 30 ml trockenem Tetrahydrofuran zu. Man läßt 10 h bei dieser Temperatur rühren, saugt vom Niederschlag ab und dampft zur Trockne ein. Der Rückstand (8.4 g) wird in 150 ml Methylenchlorid gelöst und 3 mal mit je 150 ml Wasser extrahiert. Die wäßrigen Phasen werden mit Natriumchlorid gesättigt und 3 mal mit je 150 ml Ethylacetat extrahiert. Die Ethylacetat-Phasen werden getrocknet und eingedampft. Der Rückstand (3.9 g) wird in 30 ml Ethylacetat gelöst und mit 1.59 g Cyclaminsäure in 60 ml Ethylacetat versetzt. Die abgeschiedenen Kristalle werden abgesaugt und getrocknet.

Man erhält 1.8 g (13% d.Th.) Kristalle vom Schmelzpunkt 118/120°C.

Die als Ausgangsmaterial benoetigte Verbindung 1-(4-Amino-2-ethoxycarbonyl)phenoxy-3-tert.-butyl-

amino-2-propanol wird nach an sich bekannten Verfahren und gemaeß den Vorschriften in EP 29 992 hergestellt.

Beispiel 8

1-[4-[(3-Nitroxypropyl)amino]carbonylmethyl]phenoxy-3-tert.-butylamino-2-propanol fumarat

Zu 13.4 g 2,3-Epoxy-1-[4-[(3-nitroxypropyl)amino]carbonylmethyl]phenoxy-propan gibt man 26.5 ml tert.-Butylamin und ruehrt 15 h bei Raumtemperatur. Das ueberschuessige Amin wird in Vakuum abgezogen, der Rueckstand in 150 ml Methylenchlorid geloest, 2 mal mit je 30 ml gesaettigter Natriumchlorid-Loesung, dann 2 mal mit je 50 ml 0.01 N-Salzsaeure und nochmals mit 30 ml gesaettigter Natriumchlorid-Loesung gewaschen, mit Aktivkohle gereinigt, getrocknet und eingeengt. Der verbliebende Rueckstand (12.8 g) wird in 150 ml Ethylacetat geloest und mit einer Loesung von 3.9 g Fumarsaeure in 200 ml Aceton versetzt. Das sich abscheidende Oel wird dekantiert, mit 200 ml Diethylether versetzt und 12 h bei Raumtemperatur geruehrt. Das kristallisierte Salz wird abgesaugt und getrocknet.

Man erhaelt 13.2 g (61% d.Th.) Kristalle vom Schmelzpunkt 98—100°C.

Beispiel 9

In analoger Weise, wie in Beispiel 8 beschrieben, erhaelt man aus 2,3-Epoxy-1-[4-[(Nitroxyalkyl)amino]carbonylmethyl]phenoxy-propanen und primaeren Aminen folgende Verbindungen:

| Bezeichnung | Ausb. % | Schmelzpunkt °C<br>Lösungsmittel |
|---|---|---|
| a) 1-[4-[(2-Nitroxypropyl)amino]carbonylmethyl]-phenoxy-3-tert.-butylamino-2-propanol hemifumarat<br>aus<br>2,3-Epoxy-1-[4-[(2-nitroxypropyl)amino]-carbonylmethyl]phenoxy-propan und<br>tert.-Butylamin | 50 | 78—79<br>(Diethylether) |
| b) 1-[4-[(2,2-Dimethyl-3-nitroxypropyl)amino]-carbonylmethyl]phenoxy-3-tert.-butylamino-2-propanol fumarat<br>aus<br>2,3-Epoxy-1-[4-[(2,2-dimethyl-3-nitroxy-propyl)amino]carbonylmethyl]phenoxy-propan und<br>tert.-Butylamin | 60 | 180—182<br>(Diethylether) |
| c) 1-[4-[[N-(2,2-Dimethyl-3-nitroxypropyl)-N-methyl]amino]carbonylmethyl]phenoxy-3-tert.-butylamino-2-propanol-fumarat<br>aus<br>1-[4-[[N-(2,2-Dimethyl-3-nitroxypropyl)-N-methyl]amino]carbonylmethyl]phenoxy-2,3-epoxy-propan und<br>tert.-Butylamin | 57 | 140—142<br>(Aceton) |

| | Bezeichnung | Ausb. % | Schmelzpunkt °C Lösungsmittel |
|---|---|---|---|
| d) | trans-1-[4-[(2-Nitroxycyclohexyl)amino]-carbonylmethyl]phenoxy-3-tert.-butyl-amino-2-propanol<br><br>aus<br><br>trans-2,3-Epoxy-1-[4-[(2-nitroxycyclo-hexyl)amino]carbonylmethyl]phenoxy-propan und<br><br>tert.-Butylamin | 26 | 103—105 (Diethylether) |
| e) | trans-1-[4-[[(2-Nitroxycyclohexyl)-methyl]amino]carbonylmethyl]phenoxy-3-tert.-butylamino-2-propanol-benzoat<br><br>aus<br><br>trans-2,3-Epoxy-1-[4-[[(4-nitroxycyclohexyl)-methyl]amino]carbonylmethyl]phenoxy-propan und<br><br>tert.-Butylamin | 18 | 110—112 (Diethylether) |
| f) | 1-[4-(4-Nitroxy-1-piperidino)carbonylmethyl]-phenoxy-3-tert.-butylamino-2-propanol fumarat<br><br>aus<br><br>2,3-Epoxy-1-[4-(4-nitroxy-1-piperidino)-carbonyl-methyl]phenoxy-propan und<br><br>tert.-Butylamin | 34 | 88—90 |
| g) | 1-[4-(3-Nitroxymethyl-1-piperidino)-carbonyl-methyl]phenoxy-3-tert.-butylamino-2-propanol fumarat<br><br>aus<br><br>2,3-Epoxy-1-[4-(3-nitroxymethyl-1-piperidino)-carbonylmethyl]phenoxypropan und<br><br>tert.-Butylamin | 47 | 88—90 (Diethylether) |
| h) | 1-[4-(4-Nitroxymethyl-1-piperidino)carbonylmethyl]phenoxy-3-tert.-butylamino-2-propanol fumarat<br><br>aus<br><br>2,3-Epoxy-1-[4-(4-nitroxymethyl-1-piperidino)-carbonylmethyl]phenoxypropan und<br><br>tert.-Butylamin | 67 | 97—99 (Diethylether) |

| Bezeichnung | | Ausb. % | Schmelzpunkt °C Lösungsmittel |
|---|---|---|---|
| i) | 3-Isopropylamino-1-[4-(4-nitroxymethyl-1-piperidino)carbonylmethyl]phenoxy-2-propanol-fumarat<br><br>aus<br><br>2,3-Epoxy-1-[4-(4-nitroxymethyl-1-piperidino)-carbonylmethyl]phenoxy-propan und<br><br>Isopropylamin | 16 | amorph |
| j) | 3-(2-Methyl-3-butin-2-yl)amino-1-[4-(4-nitroxy-methyl-1-piperidino)carbonylmethyl]phenoxy-2-propanol fumarat<br><br>aus<br><br>2,3-Epoxy-1-[4-(4-nitroxymethyl-1-piperidino)-carbonylmethyl]phenoxy-propan und<br><br>2-Methyl-3-butin-2-ylamin | 20 | 58—60 (Diethylether) |

Beispiel 10

Die als Ausgangsmaterial benoetigte Verbindung 2,3-Epoxy-1-[4-[(3-nitroxypropyl)amino]carbonylmethyl]phenoxy-propan wird wie folgt hergestellt:

Zu einer Loesung von 11.0 g 4-[(3-Nitroxypropyl)amino]carbonylmethyl-phenol in 50 ml Dimethylformamid gibt man 16.9 ml Epichlorhydrin und tropft dann bei 20—25°C 43.3 ml 1 N-Natronlauge zu. Man ruehrt noch 4 h bei Raumtemperatur und dampft dann bei 30°C Badtemperatur im Vakuum zur Trockne ein. Der Rueckstand wird mit Methylenchlorid und Wasser versetzt. Nach der Phasentrennung wird die organische Phase noch 2 mal mit Wasser gewaschen, getrocknet und eingeengt. Es verbleiben 13.4 g (100% d.Th.) eines oeligen Produktes. Dieses wird direkt weiter verarbeitet.

Beispiel 11

In analoger Weise, wie in Beispiel 10 beschrieben, wurden aus Epichlorhydrin und 4-(Nitroxyalkylamino)carbonylmethylphenolen folgende Verbindungen hergestellt:

| | Bezeichnung | Ausb. % |
|---|---|---|
| a) | 2,3-Epoxy-1-[4-[(2-nitroxypropyl)amino]carbonylmethyl]phenoxy-propan (gelbes Oel)<br><br>aus<br><br>4-[(2-Nitroxypropyl)amino]carbonylmethyl-phenol | 90 |
| b) | 2,3-Epoxy-1-[4-[(2,2-dimethyl-3-nitroxy-propyl)amino]-carbonylmethyl)phenoxy-propan (farbloses Oel)<br><br>aus<br><br>4-[(2,2-Dimethyl-3-nitroxy-propyl)amino]carbonylmethyl-phenol | 86 |
| c) | 2,3-Epoxy-1-[4-[[N-methyl-N-(2-nitroxypropyl)]amino]-carbonylmethyl]phenoxy-propan (gelbes Oel)<br><br>aus<br><br>4-[[N-Methyl-N-(2-nitroxypropyl)]amino]carbonylmethyl-phenol | 56 |

| | Bezeichnung | Ausb. % |
|---|---|---|
| d) | 1-[4-[[N-(2,2-Dimethyl-3-nitroxy-propyl)-N-methyl]-amino]carbonylmethyl]phenoxy-2,3-epoxy-propan (gelbes Oel)<br>aus<br>4-[[N-(2,2-Dimethyl-3-nitroxy-propyl)-N-methyl]-amino]carbonyl-phenol | 82 |
| e) | trans-2,3-Epoxy-1-[4-[(2-nitroxycyclohexyl)amino]-carbonylmethyl]phenoxy-propan<br>aus<br>trans-4-[(2-Nitroxycyclohexyl)amino]carbonylmethyl-phenol | 89 |
| f) | trans-2,3-Epoxy-1-[4-[[(2-nitroxycyclohexyl)methyl]-amino]carbonylmethyl]phenoxy-propan<br>aus<br>trans-4-[[(2-Nitroxycyclohexyl)methyl]amino]carbonyl-methylphenol | 100 |
| g) | 2,3-Epoxy-1-[4-(4-nitroxy-1-piperidino)carbonylmethyl]-phenoxy-propan (gelbes Oel)<br>aus<br>4-(4-Nitroxy-1-piperidino)carbonylmethyl-phenol | 99 |
| h) | 2,3-Epoxy-1-[4-(3-nitroxymethyl-1-piperidino)-carbonylmethyl]phenoxy-propan (gelbes Oel)<br>aus<br>4-(3-Nitroxymethyl-1-piperidino)carbonylmethyl-phenol | 91 |
| i) | 2,3-Epoxy-1-[4-(4-nitroxymethyl-1-piperidino)carbonyl-methyl]phenoxy-propan (farbloses Oel)<br>aus<br>4-(4-Nitroxymethyl-1-piperidino)carbonylmethyl-phenol | 100 |

Beispiel 12

Dia als Ausgangsmaterial benoetigte Verbindung 4-[(3-Nitroxypropyl)amino]carbonylmethyl-phenol wird wie folgt hergestellt:

Eine Loesung von 7.6 g (4-Hydroxyphenyl)essigsaeure in 5 ml trockenem Dimethylformamid und 200 ml trockenem Methylenchlorid wird mit 5.5 ml 4-Methylmorpholin versetzt und auf −20 bis −15°C abgekuehlt. Man tropft bei dieser Temperatur innerhalb 45 Minuten eine Loesung von 6.6 ml Chlorameisensaeure-isobutylester in 200 ml trockenem Methylenchlorid zu und ruehrt noch 1.5 h weiter. Dann gibt man bei −20 bis −15°C 9.15 g 3-Nitroxypropyl-ammonium-nitrat zu und tropft eine Loesung von 5.5 ml 4-Methyl-morpholin in 20 ml trockenem Methylenchlorid zu und ruehrt 1 h weiter. Man laeßt auf Raumtemperatur kommen und waescht nacheinander mit 55 ml 0.2 N Salzsaeure, 50 ml 5 proz. Natriumhydrogencarbonat-Loesung und 50 ml Wasser, trocknet ueber Natriumsulfat und engt ein.

Es verbleiben 11 g des oeligen Produktes (87% d.Th.).

Beispiel 13

In analoger Weise, wie in Beispiel 12 beschrieben, erhaelt man aus (4-Hydroxyphenyl) essigsaeure und Nitroxyalkyl-ammonium-nitraten folgende Verbindungen:

| | Bezeichnung | Ausb. % |
|---|---|---|
| a) | 4-[(2-Nitroxypropyl)amino]carbonylmethyl-phenol (hellgelbes Oel)<br><br>aus<br><br>2-Nitroxypropyl-ammonium nitrat | 100 |
| b) | 4-[(2,2-Dimethyl-3-nitroxy-propyl)amino]carbonylmethyl-phenol (hellgelbes Oel)<br><br>aus<br><br>(2,2-Dimethyl-3-nitroxy-propyl)ammonium nitrat | 100 |
| c) | 4-[[N-Methyl-N-(2-nitroxypropyl)]amino]carbonyl-methyl-phenol (farbloses Oel)<br><br>aus<br><br>N-Methyl-N-(2-nitroxypropyl)ammonium nitrat | 72 |
| d) | 4-[[N-(2,2-Dimethyl-3-nitroxy-propyl)-N-methyl]amino]-carbonylmethyl-phenol (gelbes Oel)<br><br>aus<br><br>N-(2,2-Dimethyl-3-nitroxy-propyl)-N-methyl-ammonium nitrat | 100 |
| e) | trans-4-[(2-Nitroxycyclohexyl)amino]carbonylmethyl-phenol<br><br>aus | 39 |
| f) | trans-4-[[(2-Nitroxycyclohexyl)methyl]amino]carbonyl-methyl-phenol<br><br>aus<br><br>trans-(2-Nitroxycyclohexyl)methyl-ammmonium nitrat | 63 |
| g) | 4-(4-Nitroxy-1-piperidino)carbonylmethylphenol (farbloses Oel)<br><br>aus<br><br>4-Nitroxy-piperidinium nitrat | 100 |
| h) | 4-(2-Nitroxymethyl-1-piperidino)carbonylmethyl)phenol (gelbes Oel)<br><br>aus<br><br>2-Nitroxymethyl-piperidinium nitrat | 100 |

| | Bezeichnung | Ausb. % |
|---|---|---|
| i) | 4-(3-Nitroxymethyl-1-piperidino)carbonylmethyl-phenol (gelbes Oel)<br>aus | 100 |
| | 3-Nitroxymethyl-piperidinium nitrat | |
| j) | 4-(4-Nitroxymethyl-1-piperidino)carbonylmethyl-phenol (gelbes Oel)<br>aus<br>4-Nitroxymethyl-piperidinium nitrat | 100 |

Die als Ausgangsmaterialien benoetigten Nitroxyalkylammonium nitrate werden nach bekannten Vorschriften (z.B. Houben-Weyl-Mueller, Methoden der organischen Chemie, Bd. VI/2, Georg Thieme Verlag, Stuttgart 1963, S. 325 ff.) aus den entsprechenden Hydroxyalkylaminen hergestellt.

**Patentansprüche**

1. Verbindungen der allgemeinen Formel I

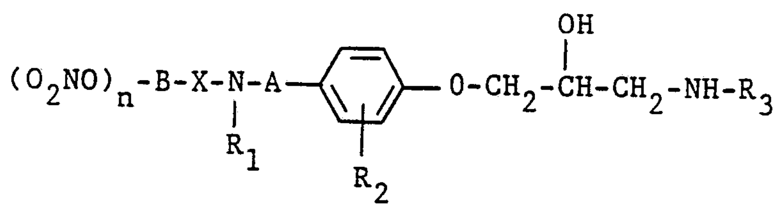

(I)

in welcher
der Rest —B—X—$NR_1$— eine ($C_2$—$C_6$-Alkylen)carbonylamino-, ($C_5$—$C_6$ cycloalkylen)carbonylamino-, Cyclohexylenmethylcarbonylamino-, Ethylenoxymethylcarbonylamino-, Piperidindiyl- oder Methylenpiperidinyl-Gruppe darstellt, oder für den Fall, daß A eine —CO—$CH_2$-Gruppe bedeutet, —B—X—$NR_1$— auch eine $C_2$—$C_6$-Alkylenaminogruppe bedeuten kann,
A eine direkte Bindung, eine Ethylenkette oder eine —CO—$CH_2$-Gruppe bedeutet,
n die Zahlen 1 bis 3 bedeutet,
$R_2$ Wasserstoff, Cyano, Acetyl oder Ethoxycarbonyl bedeutet und
$R_3$ eine Kohlenwasserstoff- oder Nitroxy-Kohlenstoff-Gruppe mit 3—8 Kohlenstoffatomen bedeutet, sowie deren physiologisch verträgliche Salze.

2. Verbindungen gemäß Anspruch 1, dadurch gekennzeichnet, daß sie
1-[4-(3-Nitroxypropyl)carbonylamino]phenoxy-3-tert.-butylamino-2-propanol
1-[4-(2-Methyl-1-nitroxy-2-propyl)-carbonylamino]phenoxy-3-tert.-butylamino-2-propanol
1-[4-[[(t-4-Nitroxy)cyclohexyl]-r-1-carbonylamino]]-phenoxy-3-tert.-butylamino-2-propanol
1-[4-[[(t-2-Nitroxy)cyclohexyl]-r-1-methylcarbonylamino]]-phenoxy-3-tert.-butylamino-2-propanol
1-[2-Cyano-4-(3-Nitroxypropyl-carbonylamino]phenoxy-3-tert.-butylamino-2-propanol
1-[2-Methylcarbonyl-4-(3-nitroxypropyl)carbonylamino]-phenoxy-3-tert.-butylamino-2-propanol
1-[2-Methylcarbonyl-4-[[(t-4-nitroxy)cyclohexyl]-r-1-carbonylamino]]phenoxy-3-tert.-butylamino-2-propanol
1-[2-Ethoxycarbonyl-4-(3-nitroxypropyl)carbonylamino]-phenoxy-3-tert.-butylamino-2-propanol
1-[4-[(2-Nitroxypropyl)amino]carbonylmethyl]phenoxy-3-tert-butylamino-2-propanol
1-[4-(4-Nitroxymethyl-1-piperidino)carbonylmethyl]-phenoxy-3-tert.-butylamino-2-propanol
bedeuten, sowie deren physioligisch verträgliche Salze.

3. Verfahren zur Herstellung von Verbindungen der allgemeinen Formel I

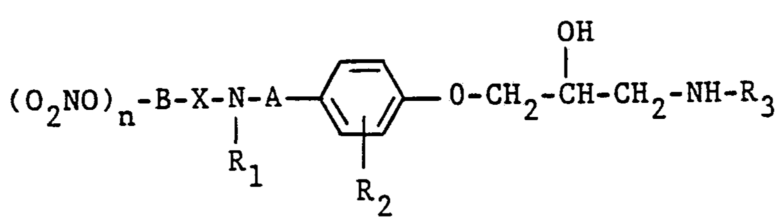

(I)

in der

der Rest $—B—X—NR_1—$ eine ($C_2—C_6$-Alkylen)carbonylamino-, ($C_5—C_6$ Cycloalkylen)carbonylamino-, Cyclohexylenmethylcarbonylamino-, Ethylenoxymethylcarbonylamino-, Piperidindiyl- oder Methylenpiperidinyl-Gruppe darstellt, oder für den Fall, daß A eine $—CO—CH_2$-Gruppe bedeutet, $—B—X—NR_1—$ auch eine $C_2—C_6$-Alkylenaminogruppe bedeuten kann,

A eine direkte Bindung, eine Ethylenkette oder eine $—CO—CH_2$-Gruppe bedeutet,

n die Zahlen 1 bis 3 bedeutet,

$R_2$ Wasserstoff, Cyano, Acetyl oder Ethoxycarbonyl bedeutet und

$R_3$ eine Kohlenwasserstoff- oder Nitroxy-Kohlenwasserstoff-Gruppe mit 3—8 Kohlenstoffatomen bedeutet,

sowie deren physiologisch verträgliche Salze, dadurch gekennzeichnet, daß man

a) eine Verbindung der allgemeinen Formel II

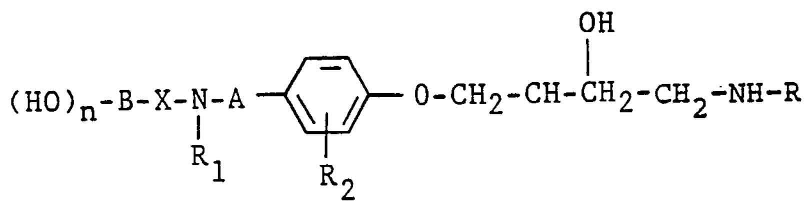 (II)

in der

A, B, X, $R_1—R_3$ und n die oben genannten Bedeutungen haben und $R_1$ zusätzlich noch die Gruppierung $—B—(OH)_n$ darstellen kann,

einer Nitratester-Bildungsreaktion unterwirft, oder

b) eine Verbindung der allgemeinen Formel III

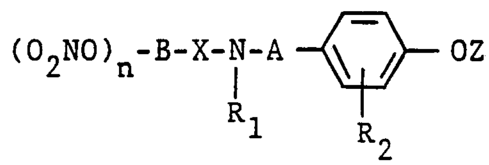 (III)

worin A, B, X, $R_1$, $R_2$ und n die oben angegebenen Bedeutungen besitzen und Z eine der Gruppierungen

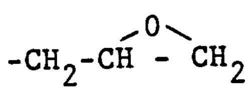 oder 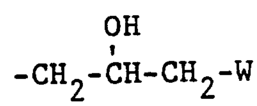

darstellt, worin W eine reaktive Gruppe bedeutet, mit einem Amin der allgemeinen Formel IV

$$H_2N—R_3 \qquad (IV),$$

in der $R_3$ die oben angegebene Bedeutung besitzt, umsetzt, oder

c) eine Verbindung der allgemeinen Formel V

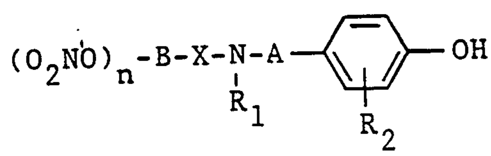 (V)

worin A, B, X, $R_1$, $R_2$ und n die oben angegebenen Bedeutungen haben

c1) mit einer Verbindung der allgemeinen Formel VI

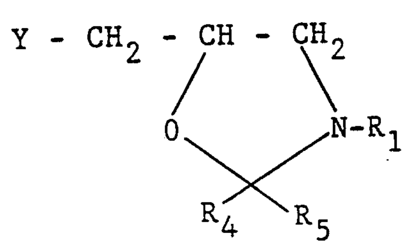 (VI)

worin $R_3$ die oben angegebene Bedeutung hat, Y Mesyloxy, Tosyloxy oder Halogen, $R_4$ Wasserstoff oder Alkyl und $R_5$ unabhaengig Wasserstoff, Alkyl oder Phenyl bedeuten oder $R_4$ und $R_5$ zusammen mit dem benachbarten Kohlenstoffatom ein Carbonyl-Radikal bilden, oder

c2) mit einer Verbindung der allgemeinen Formel VII

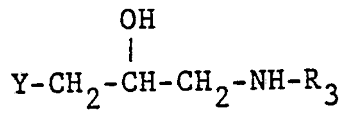

(VII)

worin Y und $R_3$ die oben angegebenen Bedeutungen haben, umsetzt, oder

d) eine Verbindung der allgemeinen Formel VIII

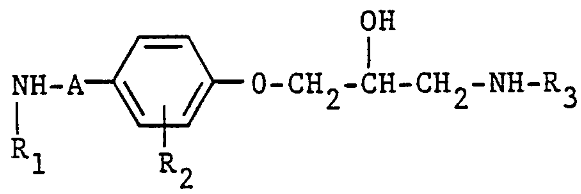

(VIII)

in der A und $R_1$—$R_3$ die oben angegebenen Bedeutungen haben, mit einer Verbindung der allgemeinen Formel IX

$$(O_2NO)_n—B—X—W \qquad (IX),$$

wobei B, W, X und n die oben angegebenen Bedeutungen haben, umsetzt, oder

e) eine Verbindung der allgemeinen Formel X

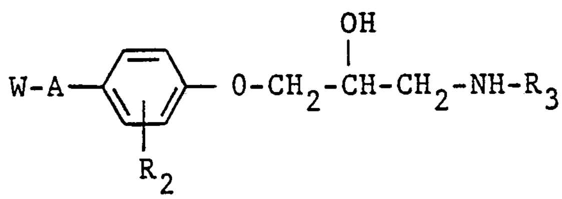

(X)

in der A, $R_2$, $R_3$ und W die oben genannten Bedeutungen haben, mit einer Verbindung der allgemeinen Formel XI

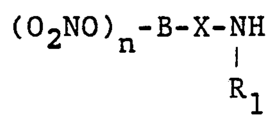

(XI)

worin B, $R_1$, X und n die oben genannten Bedeutungen haben, umsetzt und die so erhaltenen Verbindungen gewünschtenfalls in ihre physiologisch verträglichen Salze überführt.

4. Verfahren gemäß Anspruch 3 zur Herstellung der folgenden Verbindungen:
1-[4-(3-Nitroxypropyl)carbonylamino]phenoxy-3-tert.-butylamino-2-propanol
1-[4-(2-Methyl-1-nitroxy-2-propyl)-carbonylamino]phenoxy-3-tert.-butylamino-2-propanol
1-[4-[[(t-4-Nitroxy)cyclohexyl]-r-1-carbonylamino]]-phenoxy-3-tert.-butylamino-2-propanol
1-[4-[[(t-2-Nitroxy)cyclohexyl]-r-1-methylcarbonylamino]]-phenoxy-3-tert.-butylamino-2-propanol
1-[2-Cyano-4-(3-Nitroxypropyl-carbonylamino]phenoxy-3-tert.-butylamino-2-propanol
1-[2-Methylcarbonyl-4-(3-nitroxypropyl)carbonylamino]-phenoxy-3-tert.-butylamino-2-propanol
1-[2-Methylcarbonyl-4-[[(t-4-nitroxy)cyclohexyl]-r-1-carbonylamino]]phenoxy-3-tert.-butylamino-2-propanol
1-[2-Ethoxycarbonyl-4-(3-nitroxypropyl)carbonylamino]-phenoxy-3-tert.-butylamino-2-propanol
1-[4-[(2-Nitroxypropyl)amino]carbonylmethyl]phenoxy-3-tert.-butylamino-2-propanol
1-[4-(4-Nitroxymethyl-1-piperidino)carbonylmethyl]-phenoxy-3-tert.-butylamino-2-propanol.

5. Arzneimittel, gekennzeichnet durch einen Gehalt an Verbindungen gemäß Anspruch 1 oder 2 neben üblichen pharmakologisch verträglichen Träger- oder Hilfsstoffen.

6. Verwendung von Verbindungen gemäß Anspruch 1 oder 2 zur Herstellung von Arzneimitteln zur Behandlung sowie Prophylaxe von Herz- und Kreislauferkrankungen.

7. Verbindungen der allgemeinen Formel II,

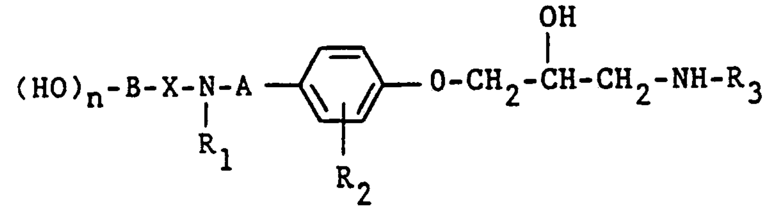

(II)

in der

der Rest —B—X—$NR_1$— eine ($C_2$—$C_6$-Alkylen)carbonylamino-, ($C_5$—$C_6$ Cycloalkylen)carbonylamino-, Cyclohexylenmethylcarbonylamino-, Ethylenoxymethylcarbonylamino-, Piperidindiyl- oder Methylenpiperidinyl-Gruppe darstellt, oder für den Fall, daß A eine —CO—$CH_2$-Gruppe bedeutet, —B—X—$NR_1$— auch eine $C_2$—$C_6$-Alkylenaminogruppe bedeuten kann,

A eine direkte Bindung, eine Ethylenkette oder eine —CO—$CH_2$-Gruppe bedeutet,

n die Zahlen 1 bis 3 bedeutet,

$R_2$ Wasserstoff, Cyano, Acetyl oder Ethoxycarbonyl bedeutet und

$R_3$ eine Kohlenwasserstoff- oder Nitroxy-Kohlenwasserstoff-Gruppe mit 3—8 Kohlenstoffatomen bedeutet.

8. Verbindungen der allgemeinen Formel III

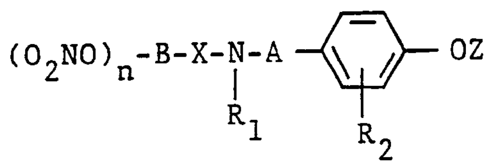

(III)

in der

der Rest —B—X—$NR_1$— eine ($C_2$—$C_6$-Alkylen)carbonylamino-, ($C_5$—$C_6$ Cycloalkylen)carbonylamino-, Cyclohexylenmethylcarbonylamino-, Ethylenoxymethylcarbonylamino-, Piperidindiyl- oder Methylenpiperidinyl-Gruppe darstellt, oder für den Fall, daß A eine —CO—$CH_2$-Gruppe bedeutet, —B—X—$NR_1$— auch eine $C_2$—$C_6$-Alkylenaminogruppe bedeuten kann,

A eine direkte Bindung, eine Ethylenkette oder eine —CO—$CH_2$-Gruppe bedeutet,

n die Zahlen 1 bis 3 bedeutet,

$R_2$ Wasserstoff, Cyano, Acetyl oder Ethoxycarbonyl bedeutet und

$R_3$ eine Kohlenwasserstoff- oder Nitroxy-Kohlenwasserstoff-Gruppe mit 3—8 Kohlenstoffatomen bedeutet, und

Z Wasserstoff oder eine der Gruppierungen

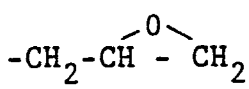 oder 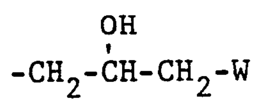

darstellt, worin W eine reaktive Gruppe bedeutet, bedeuten.

9. Verwendung von Verbindungen gemäß Anspruch 7 oder 8 zur Herstellung von Verbindungen gemäß Anspruch 1 oder 2.

**Revendications**

1. Composés de formule générale I

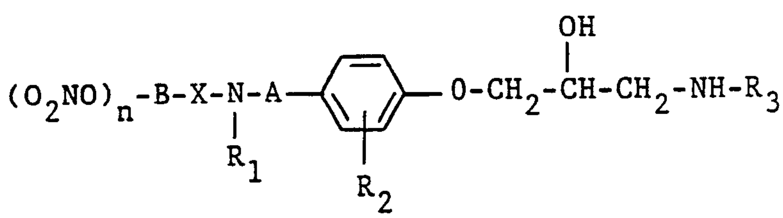

(I)

où

le reste —B—X—$NR_1$— représente un groupe alkylène($C_2$—$C_6$)-carbonylamino, cycloalkylène($C_5$—$C_6$)-carbonylamino, cyclohexylèneméthylcarbonylamino, éthylènoxyméthylcarbonylamino, pipéridinediyle ou méthylènepipéridinyle, ou dans le cas où A représente un groupe —CO—$CH_2$, —B—X—$NR_1$— peut également représenter un groupe alkylène($C_2$—$C_6$)-amino,

A représente une liaison directe, une chaîne éthylènique ou un groupe —CO—$CH_2$,
n représente un nombre de 1 à 3,
$R_2$ représente un atome d'hydrogène, un groupe cyano, acétyle ou éthoxycarbonyle, et
$R_3$ représente un groupe hydrocarbure ou nitroxy-hydrocarbure, comportant 3—8 atomes de carbone, ainsi que leurs sels physiologiquement acceptables.

2. Composés selon la revendication 1, caractérisés en ce qu'ils représentent:
1-[4-(3-Nitroxypropyl)carbonylamino]phénoxy-3-tert.-butylamino-2-propanol
1-[4-(2-Methyl-1-nitroxy-2-propyl)-carbonylamino]-phénoxy-3-tert.-butylamino-2-propanol
1-[4-[[(t-4-Nitroxy)cyclohexyl]-r-1-carbonylamino]]-phénoxy-3-tert.-butylamino-2-propanol
1-[4-[[(t-2-Nitroxy)cyclohexyl]-r-1-méthylcarbonylamino]]-phénoxy-3-tert.-butylamino-2-propanol
1-[2-Cyano-4-(3-Nitroxypropyl-carbonylamino]phénoxy-3-tert.-butylamino-2-propanol
1-[2-Méthylcarbonyl-4-(3-nitroxypropyl)carbonylamino]-phénoxy-3-tert.-butylamino-2-propanol
1-[2-Méthylcarbonyl-4-[[(t-4-nitroxy)cyclohexyl]-r-1-carbonylamino]]phénoxy-3-tert.-butylamino-2-propanol
1-[2-Éthoxycarbonyl-4-(3-nitroxypropyl)carbonylamino]-phénoxy-3-tert.-butylamino-2-propanol
1-[4-[(2-Nitroxypropyl)amino]carbonylméthyl]phénoxy-3-tert.-butylamino-2-propanol
1-[4-(4-Nitroxyméthyl-1-pipéridino)carbonylméthyl]-phénoxy-3-tert.-butylamino-2-propanol
ainsi que leurs sels physiologiquement acceptables.

3. Procédé pour la préparation des composés de formule générale I

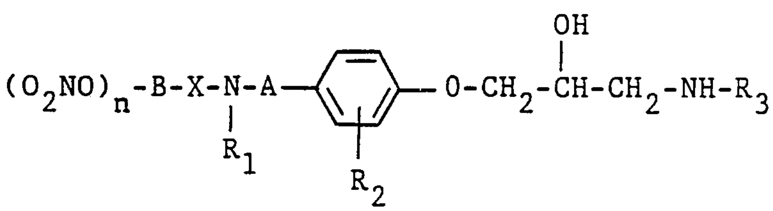

(I)

où
le reste —B—X—$NR_1$— représente un groupe alkylène($C_2$—$C_6$)-carbonylamino, cycloalkylène($C_5$—$C_6$)-carbonylamino, cyclohexylèneméthylcarbonylamino, éthylènoxyméthylcarbonylamino, pipéridinediyle ou méthylènepipéridinyle, ou dans le cas où A représente un groupe —CO—$CH_2$, —B—X—$NR_1$— peut également représenter un groupe alkylène($C_2$—$C_6$)-amino,
A représente une liaison directe, une chaîne éthylènique ou un groupe —CO—$CH_2$,
n représente un nombre de 1 à 3,
$R_2$ représente un atome d'hydrogène, un groupe cyano, acétyle ou éthoxycarbonyle, et
$R_3$ représente un groupe hydrocarbure ou nitroxy-hydrocarbure, comportant 3—8 atomes de carbone, ainsi que leurs sels physiologiquement acceptables, caractérisé en ce que
a) on soumet un composé de formule II

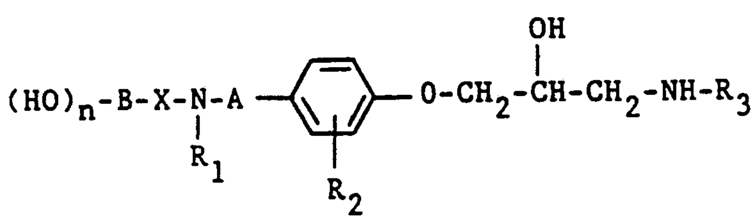

(II)

où
A, B, X, $R_1$—$R_3$ et ont les significations mentionnées plus haut, et $R_1$ peut représenter en outre le groupe —B—$(OH)_n$,
à une réaction de formation de nitrate-ester, ou
b) on fait réagir un composé de formule générale III

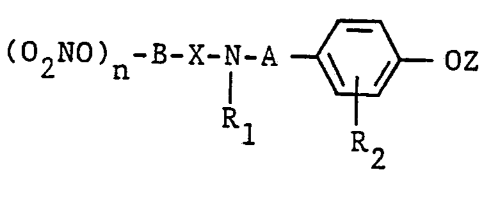

(III)

où
A, B, X, $R_1$, $R_2$ et n ont les significations mentionnées plus haut, et Z représente un groupe

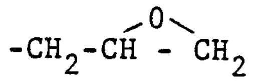 or 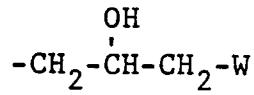

W représentant un groupe réactif, avec une amine de formule générale IV

$$H_2N—R_3 \quad (IV),$$

où $R_3$ a la signification mentionnée plus haut, ou

c) on fait réagir un composé de formule générale V

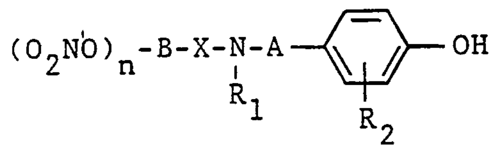 (V)

où

A, B, X, $R_1$, $R_2$ et n ont les significations mentionnées plus haut,

c1) avec un composé de formule générale VI

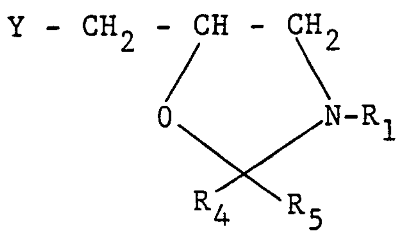 (VI)

$R_3$ a la signification mentionnée plus haut, Y représente un groupe mésyloxy, tosyloxy ou halogène, $R_4$ représente un atome d'hydrogène ou un groupe alkyle et $R_5$ représente indépendamment un atome d'hydrogène, un groupe alkyle ou phényle ou $R_4$ et $R_5$ forment, conjointement avec l'atome de carbone contigu, un radical carbonyle, ou

c2) avec un composé de formule générale VII

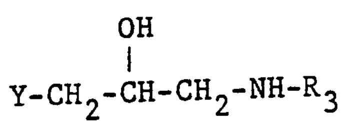 (VII)

où

Y et $R_3$ ont les significations mentionnées plus haut, ou

d) on fait réagir un composé de formule générale VIII

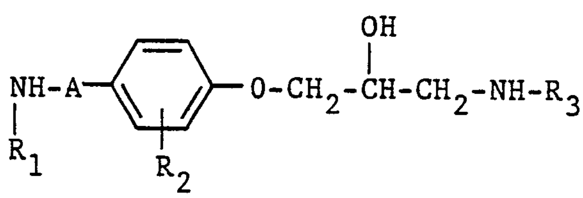 (VIII)

où

A et $R_1$—$R_3$ ont les significations mentionnées plus haut, avec un composé de formule générale IX

$$(O_2NO)_n—B—X—W \quad (IX),$$

où

B, W, X et n ont les significations mentionnées plus haut, ou

e) on fait réagir un compose de formule générale X

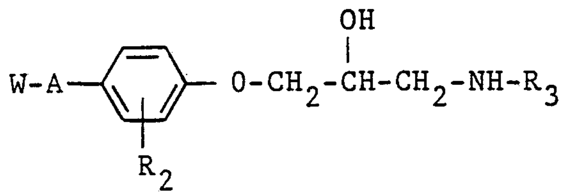 (X

où

A, $R_2$, $R_3$ et W ont les significations mentionnées plus haut, avec un composé de formule générale XI

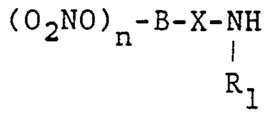 (XI)

où

B, $R_1$, X et n ont les significations mentionnées plus haut,

et on transforme éventuellement les composés ainsi obtenus en leurs sels physiologiquement compatibles.

4. Procédé selon la revendication 3, pour la préparation des composés suivants:

1-[4-(3-Nitroxypropyl)carbonylamino]phénoxy-3-tert.-butylamino-2-propanol

1-[4-(2-Methyl-1-nitroxy-2-propyl)-carbonylamino]phénoxy-3-tert.-butylamino-2-propanol

1-[4-[[(t-4-Nitroxy)cyclohexyl]-r-1-carbonylamino]]-phénoxy-3-tert.-butylamino-2-propanol

1-[4-[[(t-2-Nitroxy)cyclohexyl]-r-1-méthylcarbonylamino]]-phénoxy-3-tert.-butylamino-2-propanol

1-[2-Cyano-4-(3-Nitroxypropyl-carbonylamino]phénoxy-3-tert.-butylamino-2-propanol

1-[2-Méthylcarbonyl-4-(3-nitroxypropyl)carbonylamino]-phénoxy-3-tert.-butylamino-2-propanol

1-[2-Méthylcarbonyl-4-[[(t-4-nitroxy)cyclohexyl]-r-1-carbonylamino]]phénoxy-3-tert.-butylamino-2-propanol

1-[2-Éthoxycarbonyl-4-(3-nitroxypropyl)carbonylamino]-phénoxy-3-tert.-butylamino-2-propanol

1-[4-[(2-Nitroxypropyl)amino]carbonylméthyl]phénoxy-3-tert.-butylamino-2-propanol

1-[4-(4-Nitroxyméthyl-1-pipéridino)carbonylméthyl]-phénoxy-3-tert.-butylamino-2-propanol.

5. Produit pharmaceutique caractérisé par une teneur en composés selon la revendication 1 ou 2, en plus des adjuvants et supports pharmacologiquement acceptables usuels.

6. Utilisation des composés selon le revendication 1 ou 2, pour la préparation de produits pharmaceutiques ainsi que pour la prophylaxie des maladies cardiovasculaires.

7. Composés de formule générale II

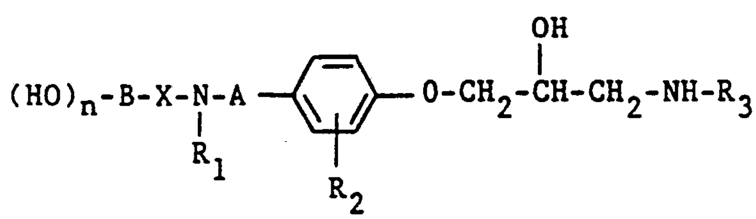 (II)

où

le reste $—B—X—NR_1—$ représente un groupe alkylène($C_2—C_6$)-carbonylamino, cycloalkylène($C_5—C_6$)-carbonylamino, cyclohexylèneméthylcarbonylamino, éthylènoxyméthylcarbonylamino, pipéridinediyle ou méthylènepipéridinyle, ou dans le cas où A représente un groupe $—CO—CH_2$, $—B—X—NR_1—$ peut également représenter un groupe alkylène($C_2—C_6$)-amino,

A représente une liaison directe, une chaîne éthylènique ou un groupe $—CO—CH_2$,

n représente un nombre de 1 à 3,

$R_2$ représente un atome d'hydrogène, un groupe cyano, acétyle ou éthoxycarbonyle, et

$R_3$ représente un groupe hydrocarbure ou nitroxy-hydrocarbure, comportant 3—8 atomes de carbone.

8. Composés de formule générale III

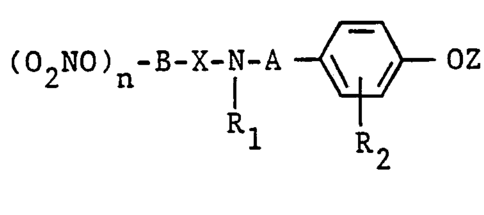 (III)

où

le reste $—B—X—NR_1—$ représente un groupe alkylène($C_2—C_6$)-carbonylamino, cycloalkylène($C_5—C_6$)-

carbonylamino, cyclohexylèneméthylcarbonylamino, éthylènoxyméthylcarbonylamino, pipéridinediyle ou méthylènepipéridinyle, ou dans le cas où A représente un groupe $—CO—CH_2$, $—B—X—NR_1—$ peut également représenter un groupe alkylène($C_2—C_6$)-amino,

A représente une liaison directe, une chaîne éthylènique ou un groupe $—CO—CH_2$,

n représente un nombre de 1 à 3,

$R_2$ représente un atome d'hydrogène, un groupe cyano, acétyle ou éthoxycarbonyle, et

$R_3$ représente un groupe hydrocarbure ou nitroxy-hydrocarbure, comportant 3—8 atomes de carbone, et

Z représente un atome d'hydrogène ou un groupe

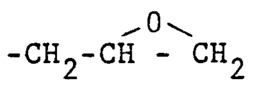 ou 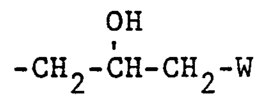

9. Utilisation des composés selon la revendication 7 ou 8, pour la préparation des composés selon la revendication 1 ou 2.

**Claims**

1. Compounds of the general formula I

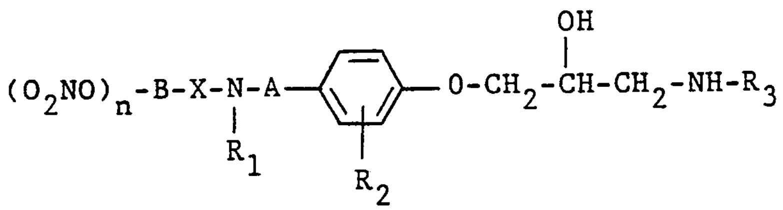 (I)

in which the radical $—B—X—NR_1—$ represents a ($C_2—C_6$-alkylene)-carbonylamino, ($C_5—C_6$-cycloalkylene)-carbonylamino, cyclohexylenemethylcarbonylamino, ethyleneoxymethylcarbonylamino, piperidinediyl or methylenepiperidinyl group or, for the case that A signifies a $—CO—CH_2—$ group, $—B—X—NR_1—$ can also signify a $C_2—C_6$-alkyleneamino group, A signifies a direct bond, an ethylene chain or a $—CO—CH_2—$ group, n signifies the numbers 1 to 3, $R_2$ signifies hydrogen, cyano, acetyl or ethoxycarbonyl and $R_3$ signifies a hydrocarbon or nitroxyhydrocarbon group with 3—8 carbon atoms, as well as their physiologically acceptable salts.

2. Compounds according to claim 1, characterised in that they signify:

1-[4-(3-nitroxypropyl)-carbonylamino]-phenoxy-3-tert.-butylamino-2-propanol

1-[4-(2-methyl-1-nitroxy-2-propyl)-carbonylamino]-phénoxy-3-tert.-butylamino-2-propanol

1-[4-[[(t-4-nitroxy)-cyclohexyl]-r-1-carbonylamino]]-phenoxy-3-tert.-butylamino-2-propanol

1-[4-[[(t-2-nitroxy)cyclohexyl]-r-1-methylcarbonylamino]]-phénoxy-3-tert.-butylamino-2-propanol

1-[2-Cyano-4-(3-nitroxypropylcarbonylamino]-phénoxy-3-tert.-butylamino-2-propanol

1-[2-méthylcarbonyl-4-(3-nitroxypropyl)-carbonylamino]-phenoxy-3-tert.-butylamino-2-propanol

1-[2-methylcarbonyl-4-[[(t-4-nitroxy)-cyclohexyl]-r-1-carbonylamino]]-phenoxy-3-tert.-butylamino-2-propanol

1-[2-ethoxycarbonyl-4-(3-nitroxypropyl)-carbonylamino]-phenoxy-3-tert.-butylamino-2-propanol

1-[4-[(2-nitroxypropyl)-amino]-carbonylmethyl]-phenoxy-3-tert.-butylamino-2-propanol

1-[4-(4-nitroxymethyl-1-piperidino)-carbonylmethyl]-phenoxy-3-tert.-butylamino-2-propanol,

as well as their physiologically acceptable salts.

3. Process for the preparation of compounds of the general formula I

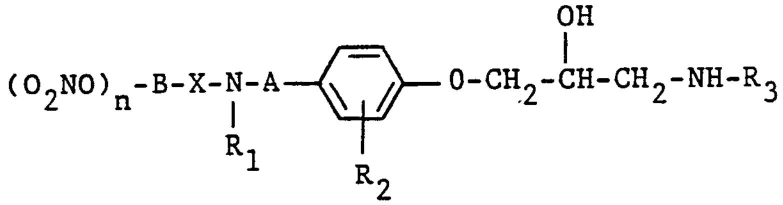 (I)

in which the radical $—B—X—NR_1—$ represents a ($C_2—C_6$-alkylene)-carbonylamino, ($C_5—C_6$-cycloalkylene)-carbonylamino, cyclohexylenemethylcarbonylamino, ethyleneoxymethylcarbonylamino, piperidinediyl or methylenepiperidinyl group or, for the case that A signifies a $—CO—CH_2—$ group, $—B—X—NR_1—$ can also signify a $C_2—C_6$-alkyleneamino group, A signifies a direct bond, an ethylene chain or a $—CO—CH_2—$ group, n signifies the numbers 1 to 3, $R_2$ signifies hydrogen, cyano, acetyl or ethoxycarbonyl and $R_3$ signifies a

hydrocarbon or nitroxyhydrocarbon group with 3—8 carbon atoms, as well as of their physiologically acceptable salts, characterised in that one

a) subjects a compound of the general formula II

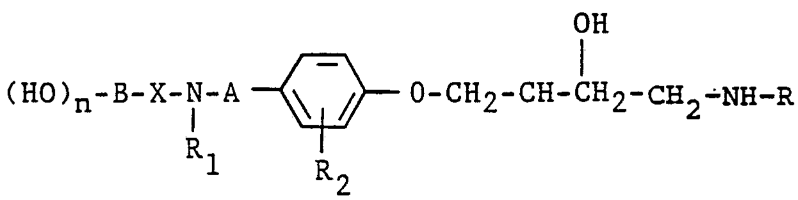

(II)

in which A, B, X, $R_1$—$R_3$ and n have the above-given meanings and $R_1$ can additionally also represent the grouping —B—$(OH)_n$, to a nitrate ester forming reaction, or

b) reacts a compound of the general formula III

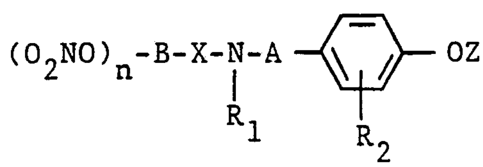

(III)

wherein A, B, X, $R_1$, $R_2$ and n have the above-given meanings and Z represents one of the groupings

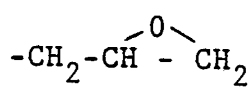

or

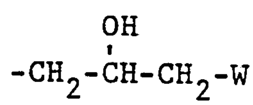

wherein W signifies a reactive group, with an amine of the general formula IV

$$H_2N—R_3 \qquad (IV)$$

in which $R_3$ possesses the above-given meaning, or

c) reacts a compound of the general formula V

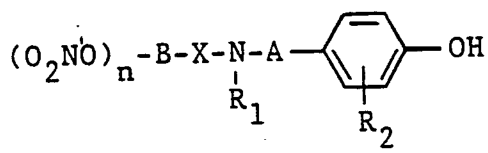

(V)

wherein A, B, X, $R_1$, $R_2$ and n have the above-given meanings

c1) with a compound of the general formula VI

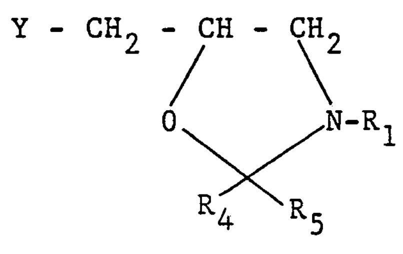

(VI)

wherein $R_3$ has the above-given meaning, Y signifies mesyloxy, tosyloxy or halogen, $R_4$ hydrogen or alkyl and $R_5$ independently hydrogen, alkyl or phenyl or $R_4$ and $R_5$, together with the neighbouring carbon atom, form a carbonyl radical, or

c2) with a compound of the general formula VII

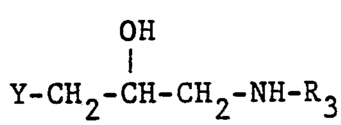

(VII)

wherein Y and $R_3$ have the above-given meanings, or

d) reacts a compound of general formula VIII

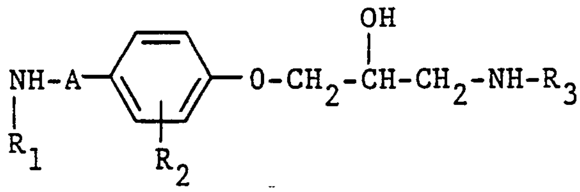

(VIII

in which A and $R_1$—$R_3$ have the above-given meanings, with a compound of the general formula IX

$$(O_2NO)_n—B—X—W \qquad (IX)$$

whereby B, W, X and n have the above-given meanings, or

e) reacts a compound of the general formula X

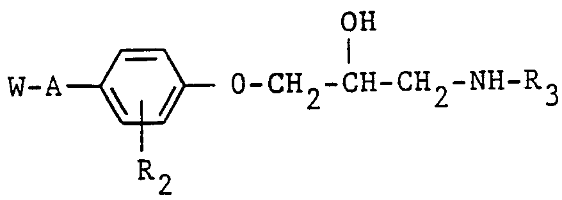

(X)

in which A, $R_2$, $R_3$ and W have the above-given meanings, with a compound of the general formula XI

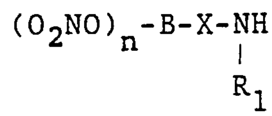

(XI)

wherein B, $R_1$, X and n have the above-given meanings, and, if desired, converts the so obtained compounds into their physiologically acceptable salts.

4. Process according to claim 3 for the preparation of the following compounds:

1-[4-(3-nitroxypropyl)-carbonylamino]-phenoxy-3-tert.-butylamino-2-propanol

1-[4-(2-methyl-1-nitroxy-2-propyl)-carbonylamino]-phénoxy-3-tert.-butylamino-2-propanol

1-[4-[[(t-4-nitroxy)-cyclohexyl]-r-1-carbonylamino]]-phenoxy-3-tert.-butylamino-2-propanol

1-[4-[[(t-2-nitroxy)cyclohexyl]-r-1-methylcarbonylamino]]-phénoxy-3-tert.-butylamino-2-propanol

1-[2-Cyano-4-(3-nitroxypropylcarbonylamino]-phénoxy-3-tert.-butylamino-2-propanol

1-[2-méthylcarbonyl-4-(3-nitroxypropyl)-carbonylamino]-phenoxy-3-tert.-butylamino-2-propanol

1-[2-methylcarbonyl-4-[[(t-4-nitroxy)-cyclohexyl]-r-1-carbonylamino]]-phenoxy-3-tert.-butylamino-2-propanol

1-[2-ethoxycarbonyl-4-(3-nitroxypropyl)-carbonylamino]-phenoxy-3-tert.-butylamino-2-propanol

1-[4-[(2-nitroxypropyl)-amino]-carbonylmethyl]-phenoxy-3-tert.-butylamino-2-propanol

1-[4-(4-nitroxymethyl-1-piperidino)-carbonylmethyl]-phenoxy-3-tert.-butylamino-2-propanol.

5. Medicaments, characterised by a content of compounds according to claim 1 or 2, besides usual pharmacologically acceptable carrier and adjuvant materials.

6. Use of compounds according to claim 1 or 2 for the preparation of medicaments for the treatment as well as prophylaxis of heart and circulatory diseases.

7. Compounds of the general formula II

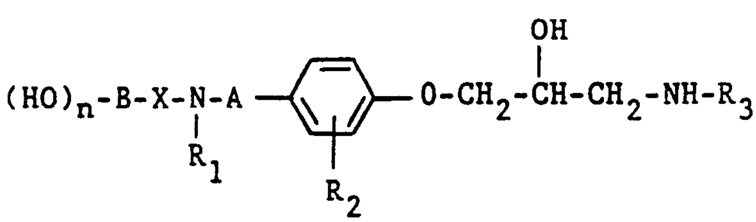

(II)

in which the radical —B—X—NR$_1$— represents a ($C_2$—$C_6$-alkylene)-carbonylamino, ($C_5$—$C_6$-cycloalkylene)-carbonylamino, cyclohexylenemethylcarbonylamino, ethyleneoxymethylcarbonylamino, piperidinediyl or methylenepiperidinyl group or, for the case that A signifies a —CO—$CH_2$— group, —B—X—NR$_1$— can also signify a $C_2$—$C_6$-alkyleneamino group, A signifies a direct bond, an ethylene chain or a —CO—$CH_2$— group, n signifies the numbers 1 to 3, $R_2$ signifies hydrogen, cyano, acetyl or ethoxycarbonyl and $R_3$ signifies a hydrocarbon or nitroxyhydrocarbon group with 3—8 carbon atoms.

8. Compounds of the general formula III

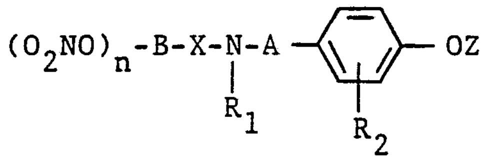

(III)

in which the radical —B—X—$NR_1$— represents a ($C_2$—$C_6$-alkylene)-carbonylamino, ($C_5$—$C_6$-cycloalkylene)-carbonylamino, cyclohexylenemethylcarbonylamino, ethyleneoxymethylcarbonylamino, piperidinediyl or methylenepiperidinyl group or, for the case that A signifies a —CO—$CH_2$— group, —B—X—$NR_1$— can also signify a $C_2$—$C_6$-alkyleneamino group, A signifies a direct bond, an ethylene chain or a —CO—$CH_2$— group, n signifies the numbers 1 to 3, $R_2$ signifies hydrogen, cyano, acetyl or ethoxycarbonyl and $R_3$ signifies a hydrocarbon or nitroxyhydrocarbon group with 3—8 carbon atoms and Z represents hydrogen or one of the groupings

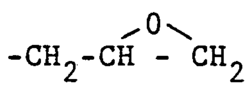 or 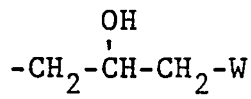

wherein W signifies a reactive group.

9. Use of compounds according to claim 7 or 8 for the preparation of compounds according to claim 1 or 2.